# FASCICULE DE BREVET EUROPEEN

(11) **EP 3 320 500 B1**
(45) Date de publication et mention de la délivrance du brevet: **08.09.2021**
(21) Numéro de dépôt: 16736151.8
(22) Date de dépôt: 06.07.2016
(51) Int. Cl.: G06Q 10/10, G06Q 50/26, G09F 3/00, G16H 10/60

(54) **DISPOSITIF ET PROCÉDÉ DE DÉCLARATION D'ÉVÈNEMENTS, D'ENREGISTREMENT DANS UN REGISTRE D'ÉTAT CIVIL, ET DE DÉLIVRANCE D'ACTES CERTIFIÉS**
VORRICHTUNG UND VERFAHREN ZUR ANMELDUNG VON EREIGNISSEN, ZUR EINTRAGUNG IN EIN GEBURTEN-, HEIRATS- UND STERBEREGISTER, UND ZUR AUSGABE ZERTIFIZIERTER OFFIZIELLER DOKUMENTE
DEVICE AND METHOD FOR DECLARING EVENTS, FOR RECORDING IN A REGISTER OF BIRTHS, MARRIAGES AND DEATHS, AND FOR ISSUING CERTIFIED OFFICIAL DOCUMENTS

(30) Priorité: 10.07.2015 FR 1556607
(43) Date de publication de la demande: 16.05.2018
(73) Titulaire: Novatec SA, 82000 Montauban (FR)
(72) Inventeur: CARABIN, François, 82000 Montauban (FR); BOURRIERES, Francis, 82000 Montauban (FR); ANDRE, Florian, 82370 Corbarieu (FR); SAWADOGO, Adama, 2148 Ouagadougou 01 (BF); KARGOUGOU, Issaka, 9772 Ouagadougou 06 (BF)
(74) Mandataire: Ipside
(86) Numéro de dépôt international: PCT/EP2016/066056
(87) Numéro de publication internationale: WO 2017/009154

(56) Documents cités:
- WO-A1-03/101041
- WO-A1-2011/016710
- FR-A1- 2 899 361
- US-A- 4 476 381
- US-A1- 2003 132 286
- US-A1- 2006 102 717

## Description

### DOMAINE

La présente invention appartient au domaine de la production d'actes certifiés concernant des individus d'une population de personnes.

Plus particulièrement l'invention concerne un dispositif et un procédé pour déclarer un événement relatif à un ou des individus, pour enregistrer officiellement cet événement sans exigence d'unité de lieu ni de temps avec la déclaration, et pour délivrer des actes certifiés relatifs à cet événement.

L'invention trouve en particulier application pour la constitution et la gestion dans le temps et dans l'espace d'un registre d'état civil d'une population, ainsi que pour la délivrance d'actes d'état civils authentiques.

### ETAT DE L'ART

Historiquement, et encore de nos jours dans certaines régions du monde, une personne n'est identifiée au sein de la population dans laquelle elle vit que par un nom, parfois un prénom, qui lui a été donné à sa naissance.

Cette méthode coutumière s'est vite révélée insuffisante avec l'augmentation de la population, la mobilité des personnes et la structuration des peuples en Etats. Un Etat a besoin pour remplir ses fonctions régaliennes de connaître la population qu'il accueille et la situation administrative de chacun des individus de sa population, en particulier pour la gestion des besoins de la collectivité, par exemple le développement des infrastructures telles que les écoles, des transports, les structures sanitaires, par exemple assurer les services exigeant la formation de personnel médical, de professeurs, de policiers. Un Etat doit également gérer les droits de ses administrés, comme par exemple le droit à l'éducation, au logement ou à diverses aides sociales.

Il a ainsi été créé dans la plupart des pays des registres d'état civil dans lesquels sont consignés les naissances des personnes et les actes considérés comme important administrativement de la vie des personnes, par exemple : baptême, mariage, décès...

Les registres d'état civil ont évolué dans leurs structures avec l'évolution des moyens techniques mais sont toujours basés sur la déclaration initiale par un parent d'une naissance auprès d'une administration ou d'une autorité administrative compétente pour recevoir la déclaration de la naissance et procéder à son enregistrement.

Dans les systèmes actuels, la déclaration de naissance doit être reçue par l'autorité compétente suivant un formalisme établi dans un délai déterminé, généralement court, après la naissance, afin en particulier d'éviter des déclarations frauduleuses.

La difficulté des systèmes actuels tient en particulier à la double exigence de lieu et de temps pour former la déclaration de naissance et, de façon consécutive, la difficulté voire l'impossibilité de procéder à l'enregistrement officiel dans un registre d'état civil.

Dans les pays disposant d'infrastructures modernes de communication et de déplacement des personnes et d'un maillage serré des administrations pouvant recevoir les déclarations de naissances, les déclarations ne posent qu'exceptionnellement problème.

Cependant dans de nombreux pays les infrastructures ne sont pas adaptées, au moins pour une partie de la population.

En effet certains Etats ne disposent que d'une structure administrative limitée et dispersée sur de vastes territoires sur lesquels vivent des populations plus ou moins isolées, ne disposant pas de moyens de communication ou de déplacement adaptés pour faire des déclarations de naissance à l'administration compétente.

Pour éviter les naissances non déclarées, parfois désignés « les enfants fantômes » par certaines publications sur le sujet, il a été mis en place des solutions pour rapprocher l'administration compétente des populations isolées. Par exemple au Nigéria, une naissance peut être signalée par un message téléphonique court (SMS) qui déclenche le déplacement d'un juge ou d'un greffier sur les lieux de la naissance, ce juge ou greffier recevant alors, en présence des autorités locales, la déclaration de la naissance et procédant à l'enregistrement et à la délivrance du certificat de naissance. (http://afrique.lepoint.fr/enfants-fantomes-un-grand-defi-pour-l-afrigue-07-05-2015-19270442254.php). Il est à noter ici que sur le plan administratif, « signalement » ne vaut pas « déclaration » et que « déclaration » ne vaut pas « enregistrement ».

A ces difficultés matérielles se superposent souvent des cultures et des modes de vie peu adaptés à la gestion des formalités administratives, et parfois de graves difficultés économiques qui repoussent au second plan les aspects formels des questions de registres de naissance.

Dans ces conditions il n'est pas rare dans certaines parties du monde que des naissances ne soient pas déclarées. Le nombre d'enfants de moins de 5 ans non déclarés est estimé par l'UNICEF à 230 millions.

Les personnes ainsi nées sans être déclarées sont alors, dans le pays où elles sont nées et vivent, sans existence légale et se trouvent le moment venu privées de l'accès à des services aussi essentiels que par exemple l'éducation ou la santé.

Il y a donc un réel besoin pour les populations et les Etats affectés par ces difficultés structurelles et ou culturelles de disposer de moyens efficaces pour construire les registres d'état civil sans qu'il leurs soit nécessaire de déployer des moyens importants, et en particulier de maîtriser la chaîne de la déclaration, de l'enregistrement et de la délivrance d'actes authentiques pour en garantir l'intégrité.

### EXPOSE DE L'INVENTION

La présente invention a précisément pour but de permettre une déclaration simple de naissance accessible à toutes les populations avec des moyens minimalistes et qui permet à l'administration de l'Etat concerné de procéder, sans contrainte d'unité dans le temps et dans l'espace, à des enregistrements et à la délivrance de documents certifiés d'état civil, avec un risque de fraude très diminué par la mise en œuvre d'éléments d'identification et d'authentification non falsifiables en pratique.

Ainsi, le système est constituer pour la déclaration d'événements relatifs à des individus ou à des groupes d'individus, l'enregistrement dans un registre d'état civil informatisé de données relatives auxdits événements et auxdits individus ou groupes d'individus, et la production de documents officiels certifiés. Le système comporte un serveur, ledit serveur comportant une base de données des données d'état civil des individus d'une population gérée par ledit serveur, comporte au moins une station terminale d'interrogation de la base de données et d'impression de documents officiels à partir des données mémorisées dans la base de données.

Le système comporte en outre au moins un terminal de déclaration et au moins un support d'identification par événement inscrit ou devant être inscrit dans ledit registre d'état civil, et dans le système :
-a) chaque support d'identification comporte au stade de sa fabrication :
   - au moins un code d'identification unique ;
   - au moins un élément authentifiant unique et non reproductible ;
   - ledit au moins un code d'identification et au moins une représentation numérique de l'au moins un élément authentifiant étant, avant d'avoir été affecté à un événement, appariés dans un registre de supports d'identification authentiques ;
-b) l'au moins un terminal de déclaration est configuré pour effectuer la déclaration d'événements à distance du lieu d'enregistrement et comporte des moyens d'acquisition du ou des codes d'identification présents sur le support d'identification, des moyens de transmission de données via un réseau de communication avec le serveur, et comporte une application logicielle :
   - d'acquisition de l'au moins un code d'identification ;
   - de saisie des données relatives à un événement relatif à un individu ou un groupe d'individus ;
   - d'élaboration d'un message court comportant l'au moins un code d'identification unique et les données saisies relatives à un événement relatif à un individu ou un groupe d'individus auquel le support d'identification, comportant l'au moins un code d'identification unique, a été ou doit être attribué ;
   - de transmission dudit message court au serveur via le réseau de communication ;
-c) ledit serveur (10) est configuré pour vérifier l'existence et la disponibilité du code d'identification reçu dans le registre (11a) des supports d'identification authentiques ;
-d) l'au moins une station terminale d'interrogation comporte une application de création de documents officiels dans laquelle au moins une représentation visuelle de l'au moins un élément authentifiant, dont au moins une représentation numérique est appariée à l'au moins un identifiant unique dans le registre de support d'identification authentiques, d'un support d'identification attribué à un individu ou un groupe d'individus, est insérée dans un document officiel relatif à l'état civil de l'individu ou d'un individu du groupe d'individus considéré.

Le système permet ainsi de transmettre les données nécessaires à la création ou la mise à jour de l'état civil d'un individu sans qu'il soit imposé à l'individu ou à des parents de se déplacer et de remplir des documents administratifs, permet également à l'administration en charge des registres d'état civil de procéder à l'enregistrement lorsque les conditions administratives sont remplies et de produire les documents officiels relatif à l'état civil d'un individu avec toutes les garanties attendues, ces étapes de déclaration, d'enregistrement et production de documents officiels étant grâce au système exonérées des exigences d'unité de temps et de lieu imposées par suivant les modes de déclaration connus..

La saisie des données est simplifiée et réalisée de préférence au moment de l'événement par une personne habilitée assistant à l'événement et qui associe la déclaration de l'événement à un support d'identification attribué à l'individu concerné par l'événement sans qu'un tiers puisse utiliser un autre support d'identification portant frauduleusement le même code d'identification pour obtenir ultérieurement des documents officiels en raison de l'élément authentifiant unique et non reproductible qui est connu préalablement par la base de données du serveur.

Un bureau d'une administration locale, plus ou moins proche du lieu de l'événement, délivre aisément à l'individu, ou aux parents dans le cas d'une naissance, un document officiel, tel qu'en extrait d'acte de naissance, pour lequel la légitimité à obtenir le document est attesté par le support d'identification porteur de l'élément authentifiant dont une image est reproduite sur le document officiel créé.

Dans une forme de réalisation, l'au moins un terminal portable mis en œuvre communique par le segment terminal sans fil d'un réseau radiotéléphonique terrestre ou satellite.

Il est ainsi fait appel à des moyens disponible dans la plupart des pays et permettant la transmission d'un faible volume de données correspondant au message court, tel que les SMS, jusque dans les lieux les plus isolés.

Dans une forme de réalisation, l'au moins un terminal portable est un téléphone-ordinateur personnel qui présente l'avantage de regrouper, dans une unité physique aujourd'hui commune et peu coûteuse, l'ensemble des fonctionnalités qui avec un support d'identification sont nécessaires et suffisantes à la personne habilitée qui sera en charge de la saisie des informations relatives à l'événement et de la transmission de ces données. Un tel terminal est en outre suffisamment simple d'utilisation pour être utilisé par une personne non spécialiste avec un minimum de formation.

Dans une forme de réalisation du dispositif, l'au moins un terminal portable incorpore un récepteur de satellites de géolocalisation pour déterminer sa position géographique. Ainsi en disposant dans le terminal portable de sa géolocalisation une position précise peut être enregistrée lors de la saisie des informations sur l'événement, par exemple au moment de la lecture optique du code d'identification du support attribué à l'individu ou au groupe d'individu. Cette donnée de localisation qui correspond en pratique au lieu de l'événement permet de vérifier, de confirmer, voire d'identifier, le lieu de l'événement si celui-ci n'a pas été saisi correctement ou simplement s'il n'est pas identifiable par un nom de lieu connu des cartes.

Dans une forme de réalisation, l'au moins élément authentifiant unique et non reproductible est choisi parmi des marqueurs, dont la formation d'une structure observable résulte d'un processus chaotique, contrôlable visuellement et/ou électroniquement, par exemple des marqueurs comportant des bulles emprisonnées dans une matrice polymère, par exemple des fibres colorées entrelacées dans la structure d'un papier.

De tels marqueurs peuvent être produits en grandes quantités dont l'unicité est physiquement garantie.

Dans une forme de réalisation l'au moins un code d'identification du bracelet est contenu dans au moins une représentation graphique lisible avec les moyens de lecture optique, par exemple dans un code graphique à deux dimensions (QR-Code).

Il est ainsi possible non seulement de procéder à une saisie du code d'identification par lecture optique du code mais en outre le code graphique à deux dimensions peut contenir d'autres informations utiles comme par exemple des adresses logiques pour adresser les messages courts au serveur, en particulier s'il n'est pas souhaité que l'adresse du serveur réside totalement dans le terminal portable, ou que cette adresse est différente sur certains supports d'identification pour des raisons techniques ou administratives par exemple.

Dans une forme de réalisation, l'au moins un code d'identification du support d'identification est contenu dans une mémoire numérique interrogeable sans contact incorporée au support d'identification.

Dans cette forme de réalisation, le code d'identification est protégé des détériorations qui pourraient rendre le code présenté sous une forme visible. En particulier, si le support d'identification a été partiellement ou totalement effacé, il pourra être lu par un lecteur adapté à tout moment y compris longtemps après la déclaration.

Avantageusement, l'au moins un code d'identification est représenté également par une chaîne de caractères alphanumériques lisible sur le bracelet. Il est ainsi possible de lire directement le code sans aucun moyen spécifique, par exemple pour une saisie au clavier lors de la recherche ou de l'édition d'un document d'état civil.

Dans une forme de réalisation, le support d'identification, au moins lorsqu'il se présente sous la forme d'un bracelet, comporte un système de fermeture non démontable sinon en détériorant ledit système de fermeture. Il est ainsi évité que le bracelet ne soit réutilisé sans que cette réutilisation ne soit détectable.

Dans une forme de réalisation, le support d'identification comporte un évidement rempli d'un matériau absorbant les liquides et comporte un volet obturateur étanche dudit évidement. Il est ainsi permis de conserver un prélèvement biologique de l'individu auquel est remis le support, par exemple une goutte de sang, qui pourra être utilisé ultérieurement pour vérifier par analyse que le support est toujours détenu par son porteur légitime.

Dans une forme de réalisation, le terminal de déclaration comporte un dispositif de capture d'une empreinte biométrique. L'empreinte capturée, transmise avec les données de l'événement, permettra également de vérifier que le porteur du support d'identification, par exemple lors de la demande d'un document officiel, est bien son détenteur légitime.

L'invention concerne également un procédé de déclaration d'événements relatifs à des individus ou à des groupes d'individus, d'enregistrement de données relatives auxdits événements et auxdits individus ou groupes d'individus dans un registre d'état civil informatisé, et de production de documents officiels certifiés relatif à l'état civil de tout individu d'une population par la mise en œuvre d'un système conforme au dispositif de l'invention.

Suivant le procédé, lors d'un événement associé à un individu ou à un groupe d'individus donné :
- un support d'identification est attribué audit individu ou à au moins un représentant dudit groupe d'individu ;
- l'au moins un code d'identification dudit support d'identification est saisi sur le terminal de déclaration, manuellement ou par des moyens de lecture dudit terminal de déclaration ;
- des données personnelles relatives audit individu ou aux individus dudit groupe d'individus, et des données relatives à l'événement sont saisies par un opérateur sur ledit terminal de déclaration, puis ;
- un message court, incorporant l'au moins un code d'identification et les données personnelles et relatives à l'événement, est généré par le terminal de déclaration puis transmis via le réseau de communication au serveur, et lesdites données sont enregistrées dans la base de données associées à l'au moins un code d'identification transmis dans le message court et à l'au moins une représentation numérique de l'au moins un élément authentifiant apparié audit au moins un code d'identification dans le registre de supports d'identification authentique, puis ;
- au moins un document est produit par une station terminale d'interrogation sur lequel document est incorporée, outre des données du registre d'état civil déclarées lors de l'événement considéré, une représentation visuelle de l'élément authentifiant dont l'au moins une représentation numériques de l'au moins un élément authentifiant est apparié audit au moins un code d'identification, attribué à l'événement lors de sa déclaration, ledit document étant déclaré document officiel lorsque le code d'identification unique et l'élément authentifiant du bracelet d'identification sont conformes respectivement au code d'identification du document produit et à ladite représentation visuelle incorporée audit document.

Il est ainsi réalisé la déclaration d'un événement affectant l'état civil d'un individu ou d'un groupe d'individus, l'enregistrement de cet événement dans le registre d'état civil, puis la création d'un document officiel d'état civil, certifié, sans exigence de déplacement et sans coût ni difficulté pour les individus concernés, par exemple pour les parents lors d'une naissance.

Dans un mode de mise en œuvre, après avoir reçu le message court, le serveur en extrait les données et l'au moins un code d'identification qu'il contient, vérifie l'existence et la disponibilité du code d'identification reçu dans le registre des supports d'identification authentiques et :
- si le code d'identification est connu et disponible, le serveur crée un enregistrement de l'événement et copie les données reçues dans la base de données d'état civil, associées à l'au moins un code d'identification et à l'au moins une représentation numérique de l'au moins un élément authentifiant apparié audit au moins un code d'identification, puis déclare ledit code d'identification correspondant attribué ;
- si le code d'identification est connu et a été déclaré attribué antérieurement, le serveur vérifie si les données reçues sont compatibles avec le ou les événements enregistrés antérieurement pour ledit code d'identification puis :
   - si lesdites données reçues sont compatibles, le serveur copie temporairement, en attente d'une étape de validation, les données reçues dans la base de données d'état civil, associées à l'au moins un code d'identification ;
   - si lesdites données reçues sont incompatibles, rejette la demande d'enregistrement.

Les données d'état civil relatives à l'événement sont ainsi liées à un support d'identification dont l'authenticité établie crée un premier niveau de certification et qui, ultérieurement, assurera le lien avec la production de documents officiels certifiés.

Dans un mode de mise en œuvre le serveur retourne au terminal de déclaration un message de réception correcte si le code d'identification est connu et disponible dans la base de données des supports d'identification authentiques, ou si les données reçues sont compatibles avec les données antérieurement associées au code d'identification attribué, et dans le cas contraire retourne audit terminal portable un message de rejet de la demande d'enregistrement au registre d'état civil.

La personne ayant transmis les données au serveur se trouve ainsi informée d'une difficulté et il est évité qu'il puisse être pensé que l'événement est inscrit à l'état civil alors qu'il ne l'est pas effectivement.

Dans un mode de mise en œuvre, le message court est crypté par le terminal de déclaration avant d'être émis, assurant ainsi un niveau de sécurité à la chaîne des données transmises.

Dans un mode de mise en œuvre, des coordonnées géographiques établies par un récepteur de données de satellites de localisation couplé au terminal de déclaration sont mémorisées par ledit terminal portable lors de la saisie du code d'identification ou des données sur l'événement et sont incorporées dans le message court envoyé au serveur.

Il est ainsi possible, sans moyen supplémentaire lorsque le terminal de déclaration incorpore, comme de nombreux ordinateurs et téléphones-ordinateurs actuels, un récepteur de satellites de localisation, de transmettre une information précise du lieu de l'événement.

Dans un mode de mise en œuvre, l'événement déclaré est une naissance et un prélèvement biologique d'un nouveau né auquel est fixé le support d'identification, tel qu'un bracelet d'identification, est déposé dans un évidement aménagé dans ledit support d'identification.

De la sorte il est permis par une analyse ultérieure du prélèvement biologique de vérifier que l'identité du porteur du support d'identification est bien celle dont les informations sont inscrites dans la base de données.

Dans un mode de mise en œuvre, il est procédé à au moins une capture d'une ou de plusieurs empreintes biométriques de l'individu auquel est attribué le support d'identification. L'enregistrement de ces empreintes biométriques dans la base de données de l'état civil apporte alors une garantie supplémentaire à la légitimité du porteur du support d'identification qui est associé, par son code d'identification et son élément authentifiant, dans la base de données aux dites empreintes biométriques.

Afin de palier à des insuffisances de couverture des réseaux sans fil, structurelle ou temporaire, en cas d'échec d'une transmission du message court, le terminal de déclaration émet à nouveau ledit message court lorsqu'il est détecté qu'une connexion au serveur est possible.

Pour la mise en œuvre du procédé et la disponibilité de supports d'identification utilisables, des supports d'identification structurellement conformes aux supports d'identification nécessaire à la mise en œuvre du procédé sont, après leur fabrication et préalablement à leur utilisation, identifiés dans un registre de supports d'identification authentiques dans lequel registre au moins un code d'identification d'un support d'identification est apparié avec au moins une représentation numérique d'un élément authentifiant incorporé audit bracelet d'identification.

Un support d'identification identifié dans le registre des supports d'identification, par ces attributs : code d'identification et représentation numérique de l'élément authentifiant, est alors déclaré authentique et peut être utilisé dans les conditions prévues.

Dans un mode de réalisation, les données reçues par le serveur et copiées temporairement dans la base de données d'état civil, associés à l'au moins un code d'identification, sont validées par un opérateur d'une station terminale d'interrogation de ladite base de données sur présentation du support d'identification portant ledit au moins un code d'identification si l'au moins un élément authentifiant porté par ledit support d'identification présente les attributs de structure des éléments authentifiant et si ledit élément authentifiant est conforme à l'au moins une représentation numérique dudit élément authentifiant appariée dans la base de données audit au moins un code d'identification.

Il est ainsi validé les données reçues sur l'événement par une action de confirmation sur présentation du support d'identification, et porté a priori par l'individu qui l'a reçu lors de l'événement.

Le procédé s'applique en particulier pour les données reçues par le serveur lors d'une déclaration qui sont relatives à un événement parmi : une naissance ; un mariage ou assimilé ; un divorce ou assimilé ; un changement de statut administratif ; un décès.

Dans un mode de mise en œuvre dans lequel l'événement est relatif à un groupe de personnes, les données relatives à l'événement, enregistrées dans la base de données des données d'état civil associées à un code d'identification, sont en outre associées, au moins temporairement en attente d'une étape de validation, à ou aux codes d'identification attribués à chacune des personnes dudit groupe connues dans ladite base de données.

Il peut ainsi être attribué un support d'identification à un événement concernant plusieurs individus, ledit support d'identification étant alors associé aux enregistrement relatifs à plusieurs personnes, ou attribués des supports d'identification, nécessairement uniques et différents, à différents individus à l'occasion d'un même événement, tout en maintenant le lien d'authenticité apporté par le ou les supports d'identification.

Dans un aspect qui ne fait pas partie de la portée de l'invention, l'exposé de l'invention se rapporte à un procédé de contrôle de l'authenticité d'un acte d'état civil édité suivant le procédé de l'invention, dans lequel au moins une image de l'élément authentifiant présentée sur ledit acte est comparée à l'image de la représentation numérique de l'élément authentifiant, supposée avoir été représentée sur ledit acte, appariée dans le registre des supports d'authentification authentiques au code d'identification associé audit acte, dont l'authenticité doit être contrôlée, ledit acte étant déclaré authentique si :
- l'au moins une image imprimée sur ledit acte correspond à l'image de la représentation numérique de l'élément authentifiant dans le registre des supports d'authentification authentiques ;
- toutes les données imprimées sur ledit acte correspondent aux données dans la base de données pour le code d'identification considéré ;
ledit acte étant considéré non authentique si ces conditions ne sont pas vérifiées.

Il est ainsi possible, pour une administration à laquelle est soumis un acte d'état civil présenté comme authentique, de vérifier par une interrogation à distance, depuis un terminal, de la base de données de l'état civil que le document soumis est conforme en tout point au contenu de ladite base de données.

### PRESENTATION DES FIGURES

L'invention sera mieux comprise à la lecture de la description qui suit et à l'examen des figures qui l'accompagnent. Celles-ci sont données à titre indicatif dans une application de déclaration de naissance et nullement limitatif de l'invention. Les figures montrent de manière schématique :
- Figure 1 : une vue d'un dispositif pour l'enregistrement des naissances dans un registre d'état civil et pour la délivrance d'actes authentiques ;
- Figure 2 : une vue d'un exemple de bracelet d'identification suivant l'invention à fixer au poignet d'un nouveau né avec une vue agrandie de la zone comportant les identifiants et les moyens d'authentification, détail (a) ; une illustration d'un exemple d'élément authentifiant du type code à bulles, détail (b) ;

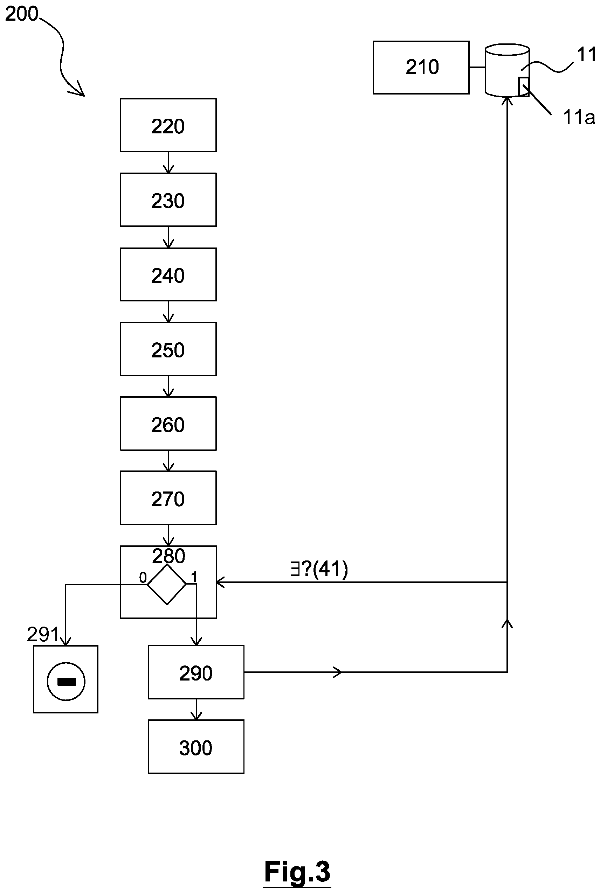

- Figure 3 : un synoptique du procédé par lequel il est créé un enregistrement d'une naissance dans le registre de l'état civil ;
- Figures 4a à 4g : des exemples d'affichages de l'écran d'un terminal du type téléphone-ordinateur à différentes étapes du processus de saisie et de validation des données nécessaires à l'enregistrement d'une naissance dans le registre de l'état civil ;
- Figure 5 : un exemple d'acte authentique « extrait d'acte de naissance » résultant de l'enregistrement d'une naissance avec le dispositif et suivant le procédé de l'invention.

### DESCRIPTION DETAILLEE D'UN MODE DE REALISATION

L'invention est décrite suivant un mode de réalisation principal dans l'exemple de la déclaration de naissances.

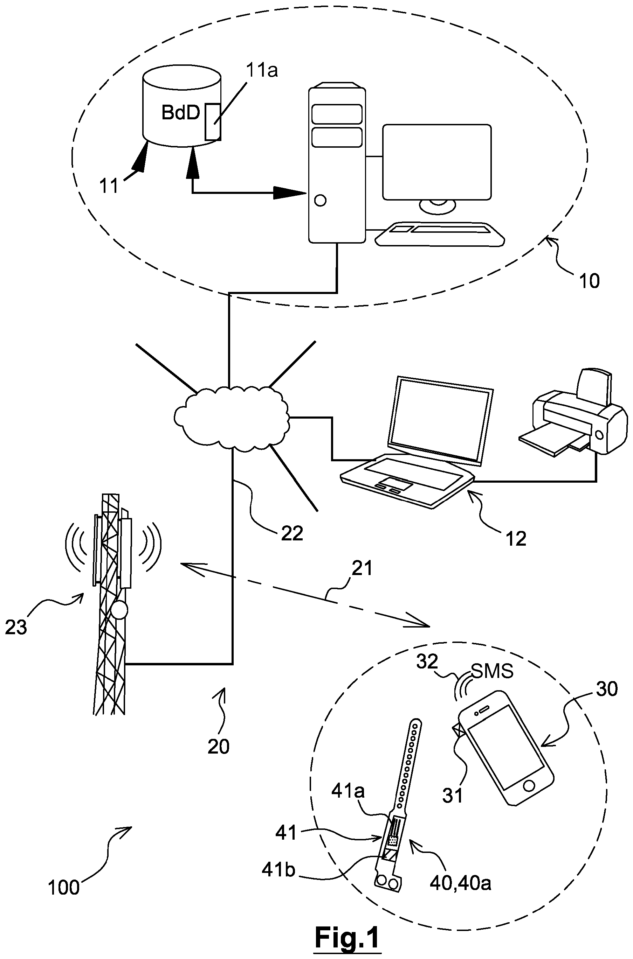

La figure 1 illustre de manière schématique les principaux composants physiques qui sont mis en œuvre par le système 100 de l'invention pour la déclaration d'événements et la gestion d'enregistrements d'un registre d'état civil et la production de documents officiels ou d'actes certifiés.

Le système 100 de la figure 1 comporte :
- un serveur 10 de données associé à une base de données 11 ;
- au moins un terminal 30 de déclaration, mis en œuvre pour réaliser la déclaration d'un événement relatif à un individu ou un groupe d'individus ;
- un réseau de communication 20 par lequel l'au moins un terminal 30 de déclaration et le serveur 10 peuvent communiquer ;
- au moins une station terminale 12 d'interrogation de la base de données et d'impression de documents officiels, comme par exemple un extrait d'acte de naissance ;
- au moins un bracelet authentique 40a, correspondant à une forme de réalisation d'un support d'identification, en pratique une pluralité de bracelets authentique en fonction du nombre d'événements devant être enregistrés. Un bracelet authentique 40a est un bracelet d'identification 40, dont un exemple de réalisation sera décrit ultérieurement qui a été référencé dans le système 100.

Le serveur 10 de données, représenté sur la figure 1 sous une forme unitaire, est un ensemble informatique conventionnel dans le domaine des serveurs de données et comportant les applications nécessaires à la création d'enregistrements dans la base de données 11, à leurs structurations, à leurs sauvegardes, à leurs lectures...

Le serveur 10 de données peut également être formé de plusieurs ordinateurs ou être dématérialisé dans le contexte de la toile d'un réseau de communication mettant en relations des ressources informatiques distantes.

Le serveur 10 de données est interconnecté autant que de besoins avec d'autres moyens informatiques en particuliers avec la station terminale 12 d'interrogation de la base de données et d'impression de documents officiels.

Le serveur 10 de données est également connecté à un serveur de messages courts, comme par exemple les messages alphanumériques désignés SMS, non représenté sur les figures, duquel il reçoit les déclarations. Le serveur de message court est par exemple raccordé au réseau de communication 20. Dans une autre forme de réalisation, le serveur de message court est intégré avec le serveur 10 de données.

En pratique le système comporte autant de stations terminales que nécessaire pour permettre la production de documents officiels en tous lieux et à tous moments souhaités.

De telles architectures informatiques sont aujourd'hui conventionnelles et il existe déjà des registres d'état civil informatisés disposant d'une banque de données centrale qui peut être interrogées par des terminaux depuis des centres administratifs plus ou moins distants pour imprimer des certificats de naissance par exemple.

Ces différents aspects techniques connus, ou a venir, du serveur, qui peuvent prendre des formes très variables, ne seront pas plus détaillés dans la présente demande.

Dans une forme de réalisation, le réseau de communication 20 comporte au moins un segment terminal 21 de communication sans fil, typiquement un réseau de téléphonie cellulaire sans fil du type GSM comme illustré sur la figure 1, ou un réseau de télécommunication par satellite, correspondant à la technologie du terminal 30 de déclaration, portable dans ce cas, qui sera utilisé pour la transmission de données depuis ledit terminal de déclaration vers le serveur 10.

Dans d'autres formes de réalisation, le terminal 30 de déclaration est raccordé au réseau 20 par des liaisons filaires ou des liaisons sans fil locales, radio ou infrarouge par exemple.

Le choix d'une forme de terminal sera en pratique dicté par les capacités de connexion au réseau dans la ou les zones dans lesquelles le terminal doit opérer. Avantageusement, le terminal 30 de déclaration est un équipement de type téléphone ordinateur, ordinateur personnel ou tablette informatique. Avantageusement, le terminal 30 de déclaration, lorsqu'il est itinérant, comporte des moyens de connexion de différentes formes lui permettant de se connecter au réseau en fonction des capacités locales dans lesquelles il doit opérer.

Le réseau de communication 20 comporte également autant que de besoin un segment intermédiaire 22 adapté au transport des données, depuis des antennes 23 ou autres équipements de routage raccordés à l'au moins un segment terminal 21 et au travers du serveur de messages courts SMS, vers le serveur 10. Le segment intermédiaire 22 peut comporter tout type de moyens de transmission connus ou à venir et de leurs combinaisons, par exemple liaisons par fils, par fibres optiques, par radio terrestre, par radio satellite.

Comme il sera compris de la suite de la description, les exigences en performances du réseau de communications 20 sont, pour les besoins du système 100 de l'invention, particulièrement limitées et à la portée de la quasi totalité des systèmes de communication téléphoniques existants.

Le terminal 30 de déclaration, étant entendu que le système comporte autant de terminaux que nécessaire, par exemple que de personnes amenées à envoyer des données par l'intermédiaire d'un tel terminal qui serait affecté à chaque personne, consiste dans l'exemple illustré en un téléphone-ordinateur personnel également désigné « smartphone ».

Comme précisé supra, le terminal 30 de déclaration peut toutefois consister en tout équipement informatique communiquant par exemple par liaison filaire, ou sans fil via des bornes de connexion, ou sans fil via un réseau de communication téléphonique de type GSM ou satellite, pour autant que ledit terminal dispose de la capacité de transmettre des messages courts.

Le terminal 30 de déclaration comporte également la capacité de saisir un code accessible sur le bracelet d'identification 40, 40a.

Avantageusement, le code est un code optique à une dimension (code à barre) ou à deux dimensions (datamatrix ou QR-Code) dont la lecture peut être réalisée par la mise en œuvre d'un capteur matriciel d'images tel que celui d'un appareil photographique ou autre moyen de lecture optique 31 intégré aux téléphones-ordinateurs et à la plupart des équipements informatiques personnels récents. Dans une autre forme de réalisation non illustrée mise en œuvre seule ou en combinaison avec d'autres formes, le code est un code radio fréquence en champ proche, généralement désigné NFC, intégré au bracelet ou toute autre technologie adaptée à délivrer un code d'identification.

Enfin le terminal 30 de déclaration comporte également une application spécifique au système, c'est à dire un logiciel spécialisé pouvant être exécuté sur ledit terminal de déclaration.

L'application, dont les fonctions principales seront expliquées au travers d'un exemple de mise en œuvre du système, est définie pour permettre à une personne habilitée de déclarer un événement, par exemple une naissance, une union, une séparation ou un décès.

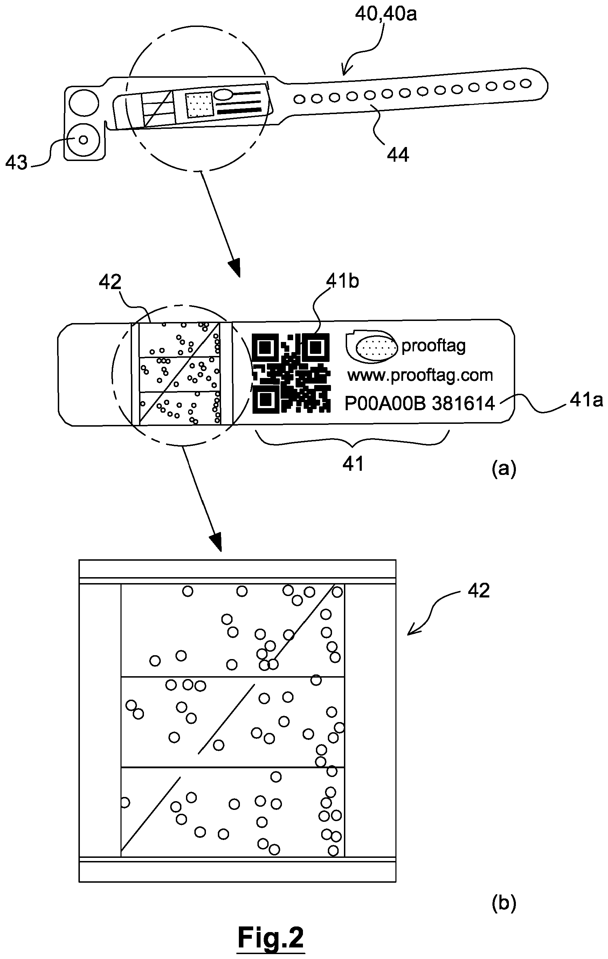

L'application permet :
- de saisir le contenu du code d'identification d'un bracelet d'identification 40, 40a, dans l'exemple du bracelet d'identification illustré sur la figure 2 contenu dans un QR-code 41b visible sur un bracelet d'identification 40, 40a de la figure 2a ;
- de saisir les données utiles pour identifier l'événement et le ou les individus concernés par ledit événement, par exemple une naissance et les informations sur un nouveau né et ses parents, et ;
- de transmettre au serveur 10, par liaison filaire ou sans fil, par exemple par radio, et via le réseau terminal 21, un message court tel qu'un message alphanumérique court (SMS) comportant l'ensemble des informations recueillies. En pratique le serveur de messages courts connecté au réseau 21 dispose de la capacité d'identifier le terminal 30 de déclaration émetteur d'un message, et de vérifier son droit d'émettre une déclaration.

Dans une forme de réalisation, ledit serveur de message court comporte le registre 11a des bracelets authentiques ou une réplication de ce registre de sorte que ledit serveur de message court vérifie l'authenticité et la disponibilité du bracelet dont le code d'identification est transmis. Si ce code est déjà utilisé, le message court est refusé.

Dans une forme de réalisation, un bracelet d'identification 40, 40a, dont un exemple est représenté sur la figure 2, est réalisé dans un matériau au contact agréable à la peau qui est de dimensions et de formes adaptées pour être placé au poignet d'une personne, par exemple d'un nouveau né lorsque l'événement est une naissance.

Des bracelets de ce type existent aujourd'hui qui comportent une surface agencée pour écrire un nom et ou un prénom d'un individu. Ces bracelets sont largement utilisés par exemple dans les maternités, en particulier pour éviter des inversions de nouveaux nés. Ces bracelets sont le plus souvent réalisés en caoutchouc naturel ou en silicone en particulier pour résister à l'eau durablement.

Le bracelet d'identification 40, 40a s'apparente à de tels bracelets connus mais présente en plus des caractéristiques essentielles spécifiques de l'invention.

Une première caractéristique est la présence sur ledit bracelet d'identification d'au moins un code d'identification 41 unique du bracelet. Ce code d'identification peut être présenté sous une ou plusieurs formes, en particulier un code alphanumérique 41a directement lisible et ou incorporé dans un code graphique tel que le QR-Code 41b du bracelet illustré pour être lu optiquement. Alternativement ou conjointement, le code d'identification peut être inscrit dans un code radio fréquence pour être lu sans contact, par exemple dans une puce RFID. Un même code d'identification peut être présent simultanément sous plusieurs formes sur le bracelet. Dans le cadre du système il est essentiel que le code puisse être saisi sur le terminal 30 de déclaration.

Le code peut être saisi par un opérateur, par exemple au moyen d'un clavier.

Le code peut également être lu par le terminal de déclaration, et pour réaliser la lecture avec des moyens ordinaires, sans nécessiter d'équipement spécialisé, il sera privilégié, dans le contexte des terminaux actuels, un code d'identification à lecture optique pouvant être lu et décrypté par un téléphone ordinateur ou autre terminal conventionnel comportant un capteur d'images.

Le code d'identification 41 est unique en ce sens qu'il n'existe pas dans le système 100 deux bracelets licites portant le même code d'identification qui lui a été attribué lors de sa fabrication.

Il doit être remarqué ici que le code d'identification est facilement reproductible et qu'il pourrait être produit illégalement un nombre quelconque de bracelets portant un même code d'identification.

Une deuxième caractéristique est la présence sur ledit bracelet d'au moins un élément authentifiant 42 unique et non reproductible. L'élément authentifiant unique 42 se distingue par une structure en trois dimensions observable.

Un élément authentifiant 42 est unique en ce sens qu'il n'existe pas deux éléments authentifiant identiques et il est non reproductible en ce sens qu'il n'est pas possible de fabriquer, au moins avec des moyens économiquement et techniquement raisonnablement accessibles, un élément authentifiant identique à un autre élément authentifiant existant.

Un tel élément authentifiant est par exemple le résultat de processus chaotiques et aléatoires produisant des hétérogénéités observables dans la matière. Un exemple d'élément authentifiant est donné par une structure de bulles emprisonnées dans un élastomère transparent comme dans le cas des « codes à bulles » diffusés par la société ProofTag ®. Un autre exemple d'élément authentifiant est donné par des fibres dispersées dans une structure de feuille de papier, colorées pour être visibles ou traitées pour être sensibles à des conditions d'éclairement spécifiques, qui forment dans la feuille de papier un motif en trois dimensions observable.

Le ou les éléments authentifiant sont solidarisés au matériau du bracelet d'identification 40 de sorte à assurer une parfaite cohésion avec ledit bracelet et de sorte que toute tentative de séparer un élément authentifiant de son bracelet ou d'en modifier l'apparence se traduise par une destruction ou une détérioration visible de l'élément authentifiant qui perd alors son caractère authentique.

L'élément authentifiant est par exemple surmoulé dans le matériau transparent, au moins localement, du bracelet 40, 40a, ou rapporté par collage ou soudage sur une face du bracelet au moyen d'un adhésif.

Dans une forme de réalisation, l'élément authentifiant est formé intégralement avec le bracelet.

Dans une forme de réalisation, le bracelet d'identification 40 comporte également un système de fermeture 43 à verrouillage définitif, c'est-à-dire que lorsqu'il a été fermé il ne peut pas être ouvert sans que ledit système de fermeture ne soit détruit ou pour le moins endommagé.

Un tel résultat est obtenu par exemple par un verrouillage en force d'un crochet non réversible, éventuellement au moyen d'une pince spécialisée, et ou de l'usage de colles ou de matériaux autosoudables.

De manière connue, le bracelet 40, 40a, comporte à une extrémité, opposée à une extrémité comportant le système de fermeture 43, une bande 44 qui coopère avec ledit système de fermeture pour fermer le bracelet avec une longueur adaptable au poignet de l'individu sur lequel il est fixé.

Une autre caractéristique du système concerne la base de données 11 qui comporte un registre 11a des bracelets authentique 40a.

Chaque bracelet d'identification 40, qui au stade de sa fabrication comporte au moins un code d'identification 41 unique et au moins un élément authentifiant 42, est enregistré dans le registre 11a de la base de données 11 par ledit au moins un code d'identification unique apparié avec au moins une représentation de l'au moins un élément authentifiant dudit bracelet.

Cette appariement de données d'un bracelet d'identification 40 dans le registre 11a, à pour effet de donner dans le système 100 une existence licite au bracelet qui devient un bracelet authentique 40a, référencé dans le système 100, dont l'unicité et l'intégrité sont le résultat des caractéristiques enregistrées et appariées dans le registre.

Il doit être noté que l'élément authentifiant, parce qu'il est unique et n'est pas reproductible, et parce qu'il est inséparable du bracelet (sans être endommagé ou détruit), apporte au bracelet le caractère unique de son code d'identification grâce à l'appairage réalisé entre le code d'identification et l'élément authentifiant dans le registre 11a de la base de données.

Le rôle de ce registre sera compris dans la description du procédé mis en œuvre par le système.

### PROCEDE

L'invention dont la structure vient d'être décrite sera mieux comprise à la lumière de la description d'un procédé 200 de mise en œuvre du système dans l'exemple de la déclaration d'une naissance, de son enregistrement dans un registre d'état civil et de délivrance d'un acte certifié.

Suivant le processus, il est mis en œuvre une étape préalable 210 d'enregistrement dans la base de données 11 de l'état civil, ou dans un registre 11a de ladite base de données ou d'une base annexe spécialement adaptée, de bracelets authentiques 40a.

Un bracelet authentique 40a est un bracelet d'identification 40, c'est-à-dire un bracelet conforme au bracelet de l'invention, comportant au moins un code d'identification unique 41 et au moins un élément authentifiant unique et non reproductible 42, enregistré dans le registre 11a. Lorsqu'il est référencé dans le registre, un tel bracelet authentique n'a pas encore été affecté à un nouveau né.

Par simplification, il sera considéré dans la suite de la description le cas d'un bracelet comportant un seul code d'identification unique et un seul élément authentifiant, ce cas particulier n'étant pas limitatif de l'invention.

L'enregistrement d'un bracelet authentique 40a consiste à appairer dans le registre 11a l'identifiant unique du dit bracelet et une ou des représentations numériques de l'élément authentifiant dudit bracelet, en particulier au moins une image sous forme numérique de l'élément authentifiant tel qu'il peut être vu sur le bracelet par un observateur, dans des conditions ordinaires ou le cas échéant à l'aide de moyens d'observation particulier comme par exemple sous un éclairage ultraviolet, le cas échéant avec des moyens de lecture spécialisés.

De tels bracelets authentiques 40a sont mis à disposition des administrations ou des personnes chargées d'accompagner les naissances, par exemple les dispensaires, les hôpitaux, les médecins, et en particulier les sages femmes qui accompagnent la plupart des accouchements.

Cette étape préalable permet d'assurer que des bracelets d'identification seront disponibles lors des naissances, au plus près de l'événement, pour permettre la déclaration d'une naissance.

Dans une première étape 220 d'affectation d'un bracelet, qui suit un accouchement, autant que possible à bref délai, un bracelet d'identification 40 est attribué au nouveau né, par exemple fixé à son poignet.

Pour des raisons pratiques, le nom donné au nouveau né peut être rapporté, écrit ou imprimé, sur le bracelet mais ceci n'est pas indispensable dans le procédé.

Ainsi le nouveau né portant un bracelet d'identification se trouve identifié par un code d'identification 40, sous forme alphanumérique 41a, et ou incorporé dans un code, par exemple un code graphique 41b, qui est associé physiquement à l'élément authentifiant unique 42 sur le bracelet qu'il porte, ainsi que dans le registre des bracelets authentiques si le bracelet est effectivement référencé dans le registre 11a suite à l'étape préalable 210.

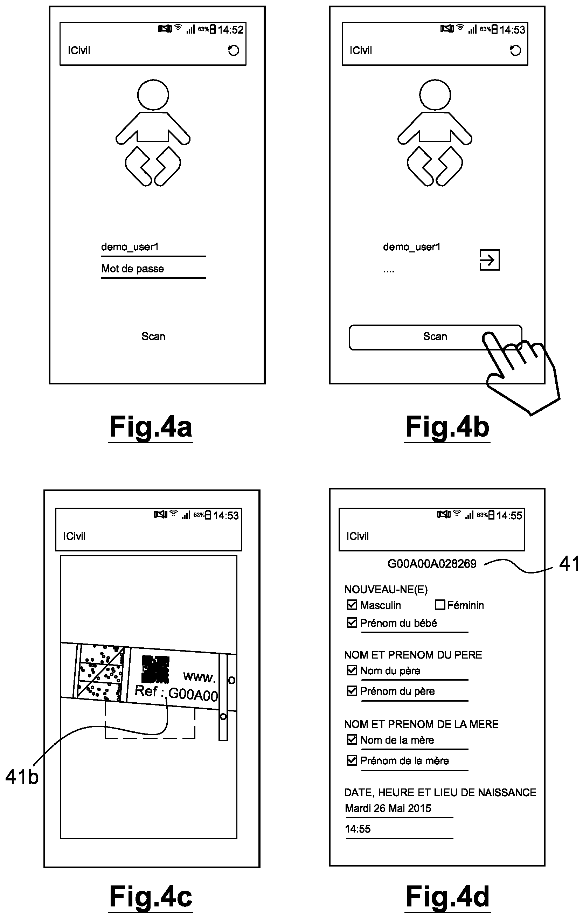

Dans une deuxième étape 230 d'activation, une personne habilitée, par exemple la sage femme ayant assisté l'accouchement, équipée d'un terminal 30 de déclaration, par exemple un téléphone-ordinateur personnel du type « smartphone » comme sur les illustrations, active sur ce terminal de déclaration l'application spécialisée pour la saisie des données de l'état civil, application dont les fonctions principales sont détaillées pour le cas d'une naissance ci-après en référence aux figures 4a à 4g reproduisant des exemples d'affichages sur un écran du terminal à chaque étape du procédé.

Pour valider le lancement de l'application, la sage femme saisit un identifiant d'utilisateur et un mot de passe qui protège l'accès à l'application, figure 4a.

Il est à noter ici que c'est l'Administration habilité qui attribue les terminaux 30 de déclaration, lesquels ne sont attribués qu'aux seules personnes qui ont Qualité en tant qu'agent assermenté à déclarer une naissance. Pour contrôler cette sécurité d'accès, un code PIN et/ou une empreinte biométrique de l'agent assermenté peuvent être utilisés.

Le système 100 a ainsi connaissance lors d'une déclaration d'au moins trois éléments fondamentaux : l'agent assermenté, le terminal de déclaration, le bracelet d'identification attribué.

Dans une troisième étape 240 de saisie du bracelet, il est procédé à la lecture du code d'identification, dans la forme illustrée un code graphique à deux dimensions 41b du type QR-Code, figure 4b et figure 4c, qui est sur le bracelet ce qui a pour conséquence de saisir le code d'identification du bracelet et qui est mis en tête des données à transmettre, figure 4d.

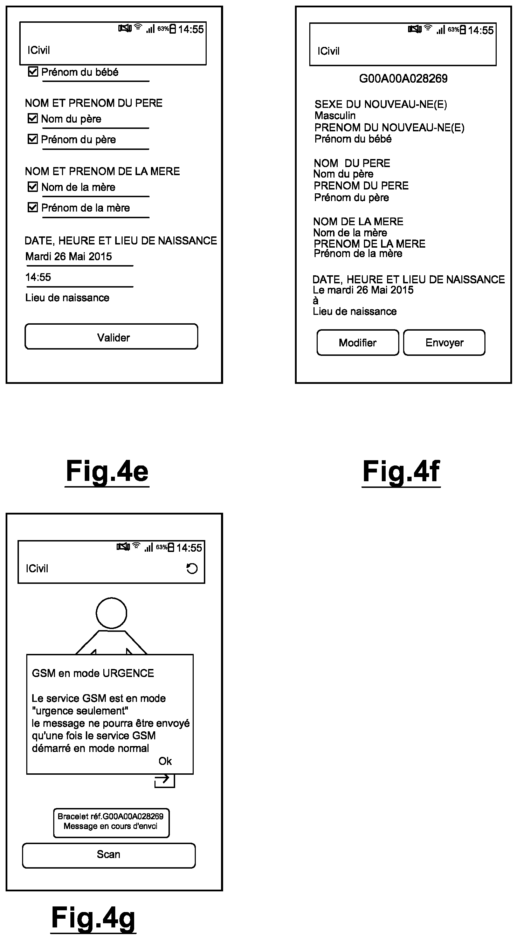

Dans une quatrième étape 250 de saisie des données de l'événement qui est enchaînée automatiquement avec la troisième étape lorsque la saisie du code, QR-Code ou autre, est validée, il est lancé une séquence de saisie des données demandées pour la création d'un enregistrement de l'état civil du nouveau né, figure 4d et figure 4e.

A ce stade du procédé, les données à saisir au travers d'un clavier sont peu nombreuses.

On trouve en particulier :
- le sexe du nouveau né
- le ou les prénoms donnés au nouveau né
- les nom et prénoms de la mère
- les nom et prénoms du père
- le lieu de naissance
- la date et l'heure de naissance

Cette saisie est par exemple réalisée au travers d'un questionnaire.

De préférence, cette saisie est effectuée peu de temps après la naissance, si elle ne peut pas être réalisée immédiatement après, pour respecter les délais administratifs de déclaration et pour obtenir les données nécessaires auprès des parents qui peuvent apporter ces données. La saisie est par exemple effectuée dans une période de 24 heures qui suit la naissance.

Dans une cinquième étape 260 de vérification, l'ensemble des données saisies est affiché en clair sur l'écran du terminal 30 de déclaration, figure 4f, pour vérifier les données saisies et donner accès à des possibilités de correction avant de donner un ordre de transmission par appui sur une touche d'envoi.

Dans une sixième étape 270 de transmission, suite à l'ordre de transmission, l'application génère un message court tel qu'un message alphanumérique court, de préférence crypté, qui est alors transmis au serveur 10 par le réseau de communication 20, 21, 22.

Dans une septième étape 280 de réception, une application, c'est à dire un logiciel spécialisé, du serveur 10, ou du serveur de messages courts, décrypte le message reçu du terminal 30 de déclaration et par une consultation du registre 11a des bracelets authentiques 40a vérifie que :
- le bracelet d'identification 40 mis au poignet du nouveau né est un bracelet dont le code d'identification 41 correspond à celui d'un bracelet authentique 40a, et ;
- que ce code n'a pas été attribué précédemment à un autre nouveau né.

Si tel n'est pas le cas, le serveur rejette, étape 291 du procédé, le message et en informe l'émetteur par un message en retour qui est affiché sur l'écran du terminal 30 de déclaration - le smartphone dans l'exemple illustré - ayant émis le message d'origine.

Si le rejet relève d'une tentative de fraude, la déclaration frauduleuse est ainsi rejetée. Dans le cas contraire le bracelet est supposé défaillant et l'étape de déclaration est reprise avec un autre bracelet authentique ou une erreur de saisie d'une donnée, par exemple l'identifiant du bracelet, est corrigée si elle est supposée à l'origine du rejet.

Si rien ne s'oppose à la prise en compte du message par le serveur, dans une huitième étape 290 de validation, le serveur 10 crée dans la base de données 11 d'état civil un nouvel enregistrement comportant l'ensemble des données relatives à l'état civil du nouveau né, transmises dans le message reçu, et associe ces données d'état civil du nouveau né, reçues avec l'identifiant unique 41 lu sur le bracelet, à l'élément authentifiant 42 unique et non reproductibles, dont la représentation numérique est associée à l'identifiant unique 41 dans la base de données des bracelets authentiques 40a.

Le bracelet authentique portant le code d'identification transmis est alors déclaré dans le registre 11a comme étant attribué au nouveau né déclaré.

A l'issue de cette séquence d'opérations, l'état civil du nouveau né est déclaré.

Il doit être remarqué dans le cas de l'invention que peu de moyens sont mis en œuvre autour du nouveau né : un bracelet d'identification, dont la forme et la taille sont communes dans l'univers des maternités, et un terminal simple tel qu'un téléphone-ordinateur ou smartphone, sur lequel est installé une application spécifique, de la personne en charge de déclarer la naissance pour assurer la transmission de messages courts, peu gourmands en volume de données à transmettre et en vitesse de transmission, compatible avec les réseaux de téléphonie actuels, avec ou sans fil, même ne disposant pas de la capacité de transmettre des données par les modes dit 3G ou 4G.

Ces moyens sont disponibles dans pratiquement tous les pays du monde, y compris dans des régions ou certaines maisons, voire certains villages ne sont pas équipés avec des téléphones filaires.

En outre, dans le cas d'un accouchement assisté dans des endroits reculés où la liaison 21 n'est pas disponible, un mode particulier de l'application mise en œuvre sur le terminal de déclaration permet de réaliser l'ensemble des opérations de saisie et de différer l'envoi du message vers le serveur, figure 4g, lorsqu'une connexion au serveur est devenue possible, par une reprise ultérieure de la sixième étape 270 de transmission.

Ainsi lorsque la personne ayant assisté l'accouchement est à nouveau dans une zone où une connexion est possible, le message est envoyé, le cas échéant de manière automatique, dès qu'une connexion au réseau est disponible.

Lorsque le serveur 10 a effectivement reçu les données et enregistré les données déclarées de l'état civil du nouveau né, de préférence il retourne un message vers le terminal ayant été utilisé de sorte que l'application sur ledit terminal cesse des tentatives de transmission des données ou informe l'utilisateur dudit terminal portable d'un échec définitif de transmission des données.

A l'issue de cette étape, sauf incident de transmission, l'ensemble des informations sur le nouveau né, nécessaires à la création d'un enregistrement dans le registre d'état civil, sont stockées dans la base de données 11 du serveur, associé par l'identifiant unique à la ou aux représentations numériques de l'élément authentifiant du bracelet attribué au nouveau né.

L'enregistrement officiel de la naissance se fera suite à la déclaration, directement ou par une opération spécifique de confirmation selon la législation en vigueur d'un état mettant en œuvre le dispositif et le procédé associé.

Par exemple, l'enregistrement officiel sera réalisé sur présentation du bracelet correspondant à un officier d'état civil qui, après contrôle d'authenticité dudit bracelet, éventuellement après enregistrement d'informations additives telle que l'adresse de résidence, procédera à l'officialisation de l'enregistrement.

Dans une neuvième étape 300 d'édition, il est créé un document officiel sur la base des données mémorisées dans la base de données 11.

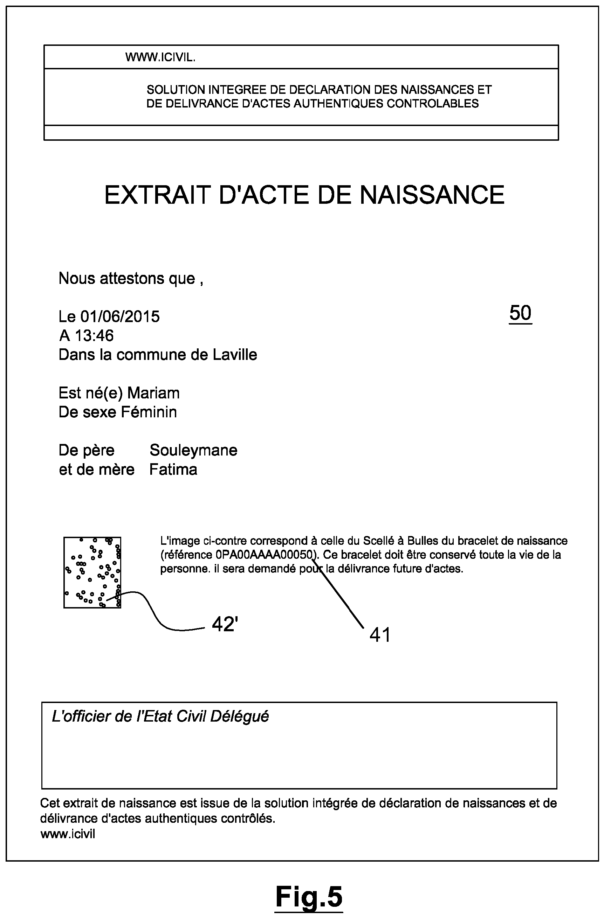

Le document officiel est par exemple un « extrait d'acte de naissance » comme dans le cas reproduit sur la figure 5.

Sur l'extrait d'acte de naissance imprimé de l'exemple illustré, sont imprimées les données traditionnelles d'un acte de naissance et en outre au moins une représentation visuelle 42' de l'élément authentifiant 42 porté par le bracelet authentique 40a associée dans la base de données aux données reproduites sur l'acte, et avantageusement le code d'identification unique 41 du bracelet apparié à ladite représentation visuelle.

Le document officiel est créé par une autorité administrative habilitée ayant accès au serveur 10 depuis une station terminale 12 d'interrogation de la base de données.

La conformité de ce document officiel avec les données déclarées et transmises au moyen du terminal de déclaration est certifiée par la présence sur ledit document officiel de la représentation visuelle 42' de l'élément authentifiant imprimé sur le document et correspondant à l'élément authentifiant 42 physique du bracelet authentique.

Le document officiel peut ainsi être produit ultérieurement à la déclaration de naissance et en tout lieu disposant des moyens et des droits pour interroger la base de données 11.

La présence du code d'identification unique 41 sur le document officiel, ou d'un autre identifiant attribué par le serveur lors de l'enregistrement des données, permet ultérieurement de consulter de manière rapide la base de données 11, par une autorité administrative habilitée, pour vérifier qu'un document officiel n'a pas été modifié ultérieurement à sa création.

L'unicité et la non reproductibilité de l'élément authentifiant 42 certifient que les données présentées sur le document officiel sont conformes à celles déclarées lors de la remise du bracelet authentique 40a.

Le document officiel peut donc être remis par l'administration, par exemple aux parents du nouveau né sous la forme d'un document imprimé, sur présentation du bracelet authentique 40a porté par le nouveau né et après avoir vérifié que l'élément authentifiant du bracelet est conforme à la ou aux représentations visuelles imprimées sur ledit document officiel.

Cette vérification est réalisée en examinant d'une part que l'élément authentifiant 42 présente bien une structure tridimensionnelle, et n'est pas une simple imitation, tel qu'une image, d'un élément authentifiant, et n'est pas endommagé, et d'autre part que l'aspect visuel de l'élément authentifiant 42 est conforme dans tous ses détails à la représentation visuelle 42' imprimée.

Ainsi, suivant le procédé et le système mis en œuvre, la déclaration d'une naissance au registre d'état civil est effectuée à distance au moment de la transmission des données lors de la déclaration de la naissance.

L'enregistrement sur le registre d'état civil est réalisé sur le constat que le bracelet d'identification utilisé est un bracelet authentique du registre et qu'il n'a pas été attribué antérieurement.

La mise en œuvre d'un bracelet authentique, ayant un identifiant unique et un élément authentifiant unique et non reproductible, assure que les données enregistrées sont celles du porteur déclaré pour le bracelet authentique considéré, et il est alors possible de délivrer un document officiel, tel qu'un acte de naissance, à tout moment ultérieurement à l'événement, acte dont l'authenticité et la légitimité sont assurées par l'élément authentifiant porté par le bracelet.

L'exemple de mise en œuvre du procédé décrit de manière détaillée dans le cas de la déclaration d'une naissance est applicable aux divers événements, appelés aussi faits d'état civil, généralement inscrits sur les registres d'état civils affectant des individus ou groupes d'individus.

Ainsi il est possible par le procédé 200 et par la mise en œuvre du système 100 de déclarer des événements tels que des unions, mariages ou équivalents, des séparations, divorces ou équivalents, des changements de nom, des décès.

La personne ayant l'autorité et les moyens de déclarer un événement n'est naturellement pas nécessairement la même pour les différentes typologies d'événement. Ainsi une naissance sera avantageusement déclarée par une sage femme ou un médecin habilité pour réaliser les déclarations de naissance et un acte de la vie civile courante le sera avantageusement une autorité administrative locale par exemple un élu, maire ou chef de village, officiant pendant l'événement déclaré.

Il est dans ces cas attribué support d'identification authentique à l'individu concerné par l'événement, ou un support d'identification différent à chaque individu lorsqu'un groupe d'individus est concerné par l'événement, ou un support d'identification à un seul individu représentant d'un groupe d'individus concernés par l'événement.

En pratique il sera mis en œuvre un support d'identification, dont le bracelet correspond à un exemple de forme ergonomique adaptée au cas des nouveaux nés déclarés, comportant au moins un code d'identification et d'au moins un élément authentifiant, support d'identification qui peut être remis à un individu, et aisément porté, lorsqu'un événement le concernant est déclaré par une autorité administrative ou une personne reconnue et autorisée par une telle autorité administrative suivant le procédé de l'invention. Par exemple le support d'identification prendra la forme d'une carte ou d'un médaillon lorsqu'il s'agit de déclarer et d'enregistrer un événement concernant une personne adulte.

Le support d'identification lorsqu'il est connu du registre 11a est donc un support d'identification authentique.

Lorsque qu'un événement déclaré a été enregistré associé à un support d'identification attribué à un individu, des données relatives à des événements successifs ultérieurs affectant le même individu peuvent également être transmises en référence à un code d'identification du support d'identification authentique détenu par ledit individu.

Le serveur 10 de telles données pour des événements ultérieurs s'assure lors de la réception des données que le nouvel événement déclaré est compatible des événements déjà enregistrés associé au code d'identification transmis et, si tel est le cas, enregistre provisoirement les nouvelles données transmises.

L'enregistrement définitif ne sera réalisé sur le serveur que lorsque la légitimité de ces données aura été prouvée à une administration disposant d'un terminal 12 d'interrogation, c'est-à-dire que le code d'identification n'a pas été usurpé. Cette légitimité sera avantageusement assurée par la présentation du support d'identification authentique.

Avantageusement, des liens logiques seront créés dans la base de données 11 entre tous les événements enregistrés pour un même individu, même dans le cas d'événements associés à différents supports d'identification authentiques.

Avantageusement, des liens logiques seront créés dans la base de données 11 entre tous les individus d'un groupe pour un même événement enregistré pour chacun des individus du groupe, même dans le cas d'événements associés à différents supports d'identification authentiques.

Dans un mode de mise en œuvre pour la production d'un document officiel, la station terminale 12 connectée au serveur 10 accède au données nécessaires à la production dudit document officiel par une lecture ou une saisie de l'identifiant unique du support d'identification détenu par le demandeur du document. La vérification de la conformité de l'élément authentifiant 42 du support d'identification à la ou aux représentations visuelles 42' imprimées sur le document démontre que le support d'identification est authentique et assure la validation administrative et la légitimité du document officiel.

Dans un autre mode de mise en œuvre pour la production d'un document officiel, la station terminale 12 connectée au serveur 10 comporte un dispositif de lecture optique de l'élément authentifiant 42 du support d'identification et accède aux données de la bases de données 11 nécessaires à la production dudit document officiel par une recherche dans ladite base de données des données associées à une ou des représentations numériques dudit élément authentifiant. Dans ce mode, la vérification de la structure et de l'intégrité de l'élément authentifiant 42 du support d'identification, et la vérification de l'identifiant unique 41 reproduit sur le document officiel avec celui du support d'identification authentique, assurent la validation administrative et la légitimité du document officiel.

Ainsi le support d'identification, tel que le bracelet authentique 40a, en particulier par son identifiant unique 41 et son élément authentifiant 42 crée le lien qui assurent l'unité de la déclaration d'un événement, de l'enregistrement de cet événement et de la délivrance d'un document officiel relatif à l'événement.

Le support d'identification authentique atteste que la personne a qui a été attribué le support d'identification authentique est bien celle qui a qui est attachée l'événement déclaré et pour laquelle il sera possible en tout lieu et à tout moment de délivrer un document administratif certifié relatif à l'événement.

Le support d'identification authentique 40a avec son élément authentifiant 42 peut être conservé par son titulaire comme il le ferait pour une carte d'identité pour se faire à tout moment reconnaître par les administrations de l'état civil ou pour la délivrance d'autres documents administratifs.

La vérification de l'authenticité d'un document, tel qu'un certificat de naissance réalisée suivant le procédé de l'invention ou un autre document administratif concernant la même personne porteur du support d'identification lui ayant été attribué à la naissance ou ultérieurement, lorsqu'il est présenté à une administration qui doit s'assurer de son authenticité, est réalisé par une consultation sur la base de données 11 du serveur des données associées au code d'identification ou un autre code administratif présent sur le document soumis et par une comparaison de la représentation numérique de l'authentifiant 42 apparié dans la base de données audit code d'identification reproduit sous la forme d'une représentation visuelle 42' sur ledit acte, ledit document étant déclaré authentique si :
- l'au moins une image 42' imprimée sur ledit document correspond à la représentation numérique de l'authentifiant 42 dans la base de données, et ;
- toutes les données imprimées sur ledit document correspondent aux données du registre dans la base de données 11 pour le code d'identification considéré ;
ledit acte étant à contrario considéré non authentique si ces conditions ne sont pas vérifiées.

L'état civil d'un nouveau né, une fois créé dans la base de données 11, peut ainsi être complété et mis à jours de tous les événements de la vie devant y figurer par tout moyen conventionnel ou avantageusement en mettant en œuvre le procédé décrit basé sur le bracelet authentique 40a, ou autre support d'identification authentique, et ses caractéristiques.

L'élément authentifiant 42 du support d'identification est le cas échéant utilisé pour authentifier l'identité de son porteur, au moins aussi longtemps qu'il n'aura pas été remplacé par une pièce d'identité qui avantageusement aura les attributs d'un support d'identification et comportera dans ce cas un élément authentifiant de même nature et lui succédera, la base de données 11 pouvant dans ce cas être mise à jour et conserver un historique des identifiants uniques et éléments authentifiant attribués à une même personne.

Dans une forme de réalisation, un support d'identification authentique comporte un évidement aménagé sur ledit support d'identification, par exemple dans l'épaisseur du bracelet sur une zone du bracelet utilisé comme support d'identification. Le volume de l'évidement est rempli d'un matériau stérile absorbant les liquides, par exemple ayant une structure en fibres de coton ou en buvard, et protégée par un film amovible.

Un tel évidement est mis en œuvre lorsqu'il est souhaité prendre une empreinte génétique de la personne à laquelle va être remis le support d'identification afin de garantir ultérieurement son identité et éviter une fraude.

Dans le cas d'une naissance, le médecin accoucheur ou la sage-femme prélève une goutte de sang du nouveau né, goutte de sang qui est déposée sur le matériau absorbant après le retrait du film amovible de protection.

Une fois cette opération réalisée, un volet obturateur de l'évidement est placé pour refermer de manière étanche le volume dudit évidement et préserver le prélèvement.

Le volet obturateur est par exemple un volet rabattable, protégé par le film amovible de protection avant d'utiliser le bracelet, et avantageusement adhésif ou autosoudable à la matière du support d'identification. Par sécurité ledit obturateur est conçu pour être détruit ou irrémédiablement endommagé en cas de tentative d'ouverture.

Le prélèvement sera ultérieurement utilisé pour établir une empreinte génétique et vérifier par comparaison qu'il est toujours bien détenu par la personne l'ayant reçu.

Dans une variante de mise en œuvre, il est procédé à une capture d'empreinte biométrique qui sera ultérieurement, par exemple lors de l'enregistrement officiel, associée au support d'identification et enregistrée dans la base de données 11. Une telle méthode est particulièrement adaptée aux adultes qui vont faire leur entrée dans le registre d'état civil.

L'invention décrite de manière détaillée pour un exemple particulier de réalisation et de mise en œuvre peut prendre d'autres formes voisines à la portée de l'homme du métier sans se démarquer des principes exposés.

En particulier les moyens techniques sont susceptibles d'évoluer et de prendre d'autres formes en continuant à assurer les fonctions nécessaires à l'invention.

Par exemple la mise en œuvre par la personne habilitée d'un téléphone-ordinateur personnel est une solution pratique compte tenu de la large diffusion de ce type d'équipement (smartphones) qui regroupe l'ensemble des fonctions de communication et de saisie nécessaires à l'application.

Une tablette-ordinateur personnelle ou un ordinateur fixe ou portable doté de capacité de communication peut également être mis en œuvre pour exécuter l'application.

Les exemples illustrés des données saisies, tant dans leurs contenus que dans leurs présentations, ne sont présentés qu'a titre d'illustration et sont susceptibles de prendre des formes et des contenus différents en fonction des moyens matériels mis en œuvre, du type d'événement déclaré et des exigences propres à chaque registre d'état civil.

Le terminal 30 de déclaration peut par exemple contenir un récepteur de localisation par satellites, GPS, GLONASS ou autre système équivalent, cas actuel de nombreux smartphones, qui délivre en particulier la latitude et la longitude du lieu ou il se trouve avec une précision de quelques dizaines de mètres. Dans ce cas l'application enregistre avantageusement ces données de localisation, par exemple au moment de la saisie de l'identifiant unique ou de la lecture optique du code 41b comportant l'identifiant unique, données qui seront transmises dans le message court envoyé au serveur. Il est ainsi possible de donner un lieu précis de l'événement lorsque celui-ci se produit loin de tout repère nommé tel qu'une ville ou un village.

Le dispositif et le procédé selon l'invention permet de lier les trois phases essentielles de déclaration, d'enregistrement officiel et de délivrance d'actes en ayant en permanence la preuve que la personne déclarée à l'instant de l'événement est bien celle qui réclame un document officiel, acte de naissance par exemple, même vingt ou trente ans plus tard, et également qu'il s'agit bien d'une même personne déclarée décédée, quel que soit son âge.

Le dispositif et le procédé de mise en œuvre permettent ainsi de réaliser des déclarations à l'état civil d'événements affectant des individus et de délivrer ultérieurement, sans contrainte de temps et de manière sécurisée, des documents officiels pour lesquels il peut être établi la conformité à l'événement bien qu'il n'ait pas été enregistré par un officier d'état civil attitré.

Il est ainsi évité les conséquences, parfois dramatiques, d'une absence de déclaration, en particulier dans des régions ne disposant pas des infrastructures nécessaires du fait d'un développement incomplet des moyens de communication et de territoires peu peuplé dans lesquels les déclarations sont problématiques et trop souvent oubliée conduisant à l'absence de déclaration en particulier de nombreux nouveaux nés par leurs parents.

L'invention apporte une solution à ce problème majeur d'une part en mettant en œuvre des moyens conventionnels sans exiger des performances élevées, en particulier en débits de transmission de données, disponibles aujourd'hui dans pratiquement tous les pays du monde, et d'autre part en reportant, avec un minimum de charge de travail, les formalités de déclaration sur une personne habilitée, tel qu'une sage femme ou un acteur local de la vie civile, équipée et formée pour réaliser la déclaration depuis le lieu de l'événement et au moment où il se produit.

Un tel système et le procédé de mise en œuvre sont donc des outils performants pour à l'avenir diminuer de manière importante le nombre de personnes vivants sans identité légale faute d'avoir été déclarées lors de leurs naissances ou sans reconnaissance officielle de leurs statuts, et pour rendre impossible toute usurpation et ou tentative de falsification. Ils permettent conséquemment aux personnes ainsi déclarées d'avoir une vie sociale légitime et d'assurer la déclaration et l'enregistrement sur le registre d'état civil de tous les événements affectant la situation administrative de la personne, par exemple un mariage, avec le même niveau d'authenticité et de légitimité que pour la déclaration de naissance.

Dans un mode de mise en œuvre, l'invention est utilisée pour des opérations de recensement des populations. Dans ce mode, le transfert de données par SMS associé à des moyens d'identification/authentification physico/numérique tel que décrits pour identifier les personnes et/ou les familles permet de renseigner directement des bases de données statistiques et ainsi de suivre en temps réel et dans l'espace les saisies effectuées par des agents recenseurs agréés disposant de terminaux de déclaration.

## Revendications

1. Système (100) pour la déclaration d'événements relatifs à des individus ou à des groupes d'individus, l'enregistrement dans un registre d'état civil informatisé de données relatives auxdits événements et auxdits individus ou groupes d'individus, et la production de documents officiels (50) certifiés, ledit système comportant un serveur (10), ledit serveur comportant une base de données (11) des données d'état civil des individus d'une population gérée par ledit serveur, comportant au moins une station terminale (12) d'interrogation de la base de données (11) et d'impression de documents officiels à partir des données mémorisées dans ladite base de données, **caractérisé en ce qu'**il comporte au moins un terminal (30) de déclaration et au moins un support d'identification par événement inscrit ou devant être inscrit dans ledit registre d'état civil, et dans lequel système :
-a) chaque support d'identification comporte au stade de sa fabrication :
- au moins un code d'identification (41, 41a, 41b) unique ;
- au moins un élément authentifiant (42) unique et non reproductible choisi parmi des marqueurs comportant une structure observable tridimensionnelle unique et non reproductible, ladite structure observable étant contrôlable visuellement et/ou électroniquement, et dont la formation de ladite structure observable résulte d'un processus chaotique et aléatoire produisant des hétérogénéités observables dans la matière ,par exemple des marqueurs comportant des bulles emprisonnées dans une matrice polymère, par exemple des fibres colorées entrelacées dans la structure d'un papier ;
- ledit au moins un code d'identification et au moins une représentation numérique de l'au moins un élément authentifiant étant, avant d'avoir été affectés à un événement, appariés dans un registre (11a) de supports d'identification authentiques, et ladite représentation numérique de l'élément authentifiant est une image sous forme numérique de l'élément authentifiant tel qu'il peut être vu par un observateur ;
-b) l'au moins un terminal (30) de déclaration est configuré pour effectuer la déclaration d'événements à distance du lieu d'enregistrement et comporte des moyens d'acquisition du ou des codes d'identification présents sur le support d'identification, des moyens de transmission de données via un réseau de communication (20) avec le serveur (10), et comporte une application logicielle :
- d'acquisition de l'au moins un code d'identification (41, 41a, 41b) ;
- de saisie des données relatives à un événement relatif à un individu ou un groupe d'individus ;
- d'élaboration d'un message court comportant l'au moins un code d'identification unique et les données saisies relatives à un événement relatif à un individu ou un groupe d'individus auquel le support d'identification, comportant l'au moins un code d'identification unique, a été ou doit être attribué ;
- de transmission dudit message court au serveur (10) via le réseau de communication (20) ;
-c) l'au moins une station terminale (12) d'interrogation comporte une application de création de documents officiels (50) dans laquelle au moins une représentation visuelle (42') de l'au moins un élément authentifiant (42), dont au moins une représentation numérique est appariée à l'au moins un identifiant unique dans le registre (11a) de support d'identification authentiques, d'un support d'identification attribué à un individu ou un groupe d'individus, est imprimée dans un document officiel relatif à l'état civil de l'individu ou d'un individu du groupe d'individus considéré, et un aspect visuel de l'élément authentifiant (42) est conforme dans tous ses détails à la représentation visuelle (42') imprimée de l'élément authentifiant.

2. Système suivant la revendication 1 dans lequel l'au moins un terminal (30) de déclaration est un terminal portable, par exemple un téléphone-ordinateur personnel, qui communique par un segment terminal (21) sans fil d'un réseau radiotéléphonique terrestre ou satellite.

3. Système suivant l'une des revendications précédentes dans lequel l'au moins un code d'identification (41) du support d'identification est représenté par une chaîne de caractères alphanumériques (41a) lisible sur le support d'identification, et/ou est contenu dans au moins une représentation graphique lisible avec les moyens de lecture optique (31), par exemple dans un code graphique à deux dimensions (QR-Code), et/ou est contenu dans une mémoire numérique interrogeable sans contact incorporée audit support d'identification.

4. Système suivant l'une des revendications précédentes dans lequel le support d'identification est un bracelet d'identification (40) qui comporte un système de fermeture (43) non démontable sinon en détériorant ledit système de fermeture.

5. Système suivant l'une des revendications précédentes dans lequel le support d'identification comporte un évidement rempli d'un matériau absorbant les liquides et comporte un volet obturateur étanche dudit évidement.

6. Système suivant l'une des revendications précédentes dans lequel le terminal (30) de déclaration comporte un dispositif de capture d'une empreinte biométrique.

7. Procédé (100) de déclaration d'événements relatifs à des individus ou à des groupes d'individus, d'enregistrement dans un registre d'état civil informatisé de données relatives auxdits événements et auxdits individus ou groupes d'individus, et de production de documents officiels (50) certifiés, ledit procédé étant mis en œuvre par un système comportant un serveur (10), ledit serveur comportant une base de données (11) des données d'état civil des individus d'une population gérée par ledit serveur, comportant au moins une station terminale (12) d'interrogation de la base de données (11) et d'impression de documents officiels à partir des données mémorisées dans ladite base de données, au moins un terminal (30) de déclaration et au moins un support d'identification par événement inscrit ou devant être inscrit dans ledit registre d'état civil, ledit procédé comprenant les étapes suivantes :
-a) une étape d'attribution d'un support d'identification audit individu ou à au moins un représentant dudit groupe d'individus lors d'un événement relatif audit individu ou audit groupe d'individus, ledit support d'identification comportant au stade de sa fabrication .
- au moins un code d'identification (41, 41a, 41b) unique ; et
- au moins un élément authentifiant (42) unique et non reproductible choisi parmi des marqueurs comportant une structure observable tridimensionnelle unique et non reproductible, ladite structure observable étant contrôlable visuellement et/ou électroniquement, et dont la formation de ladite structure observable résulte d'un processus chaotique et aléatoire produisant des hétérogénéités observables dans la matière ,par exemple des marqueurs comportant des bulles emprisonnées dans une matrice polymère, par exemple des fibres colorées entrelacées dans la structure d'un papier ;
- ledit au moins un code d'identification et au moins une représentation numérique de l'au moins un élément authentifiant étant, avant d'avoir été affectés à un événement, appariés dans un registre (11a) de supports d'identification authentiques, et ladite représentation numérique de l'élément authentifiant est une image sous forme numérique de l'élément authentifiant tel qu'il peut être vu par un observateur ;
-b) mise en œuvre de l'au moins un terminal (30) de déclaration pour effectuer la déclaration d'événements à distance du lieu d'enregistrement, et comportant des moyens d'acquisition du ou des codes d'identification présents sur le support d'identification, des moyens de transmission de données via un réseau de communication (20) avec le serveur (10), et une application logicielle agencée pour effectuer les étapes suivantes :
- acquisition de l'au moins un code d'identification (41, 41a, 41b) ;
- saisie des données relatives à un événement relatif à un individu ou un groupe d'individus, et des données personnelles relatives audit individu ou aux individus dudit groupe d'individus ;
- élaboration d'un message court comportant l'au moins un code d'identification unique, lesdites données saisies relatives à l'événement et lesdites données personnelles relatives audit individu ou aux individus dudit groupe d'individus ;
- transmission dudit message court au serveur (10) via le réseau de communication (20) ;
-c) impression, dans un document officiel relatif à l'état civil de l'individu ou d'un individu du groupe d'individus considéré, par une application de création de documents officiels (50) de l'au moins une station terminale (12) d'interrogation, outre des données du registre civil déclarées lors de l'événement considéré, d'au moins une représentation visuelle (42') de l'au moins un élément authentifiant (42), dont au moins une représentation numérique est appariée à l'au moins un identifiant unique dans le registre (11a) de support d'identification authentiques, dudit support d'identification attribué audit individu ou audit représentant dudit groupe d'individus, et dont un aspect visuel est conforme dans tous ses détails à la représentation visuelle (42') imprimée de l'élément authentifiant.

8. Procédé suivant la revendication 7, dans lequel le serveur, après avoir reçu le message court, extrait dudit message court les dites données et ledit au moins un code d'identification, vérifie l'existence et la disponibilité du code d'identification reçu dans le registre (11a) des supports d'identification authentiques et :
- si le code d'identification est connu et disponible, le serveur crée un enregistrement de l'événement et copie les données reçues dans la base de données (11) d'état civil, associées à l'au moins un code d'identification et à l'au moins une représentation numérique de l'au moins un élément authentifiant (42) apparié audit au moins un code d'identification, puis déclare ledit code d'identification correspondant attribué ;
- si le code d'identification est connu et a été déclaré attribué antérieurement, le serveur vérifie si les données reçues sont compatibles avec le ou les événements enregistrés antérieurement pour ledit code d'identification puis :
- si lesdites données reçues sont compatibles, le serveur copie temporairement, en attente d'une étape de validation, les données reçues dans la base de données (11) d'état civil, associées à l'au moins un code d'identification ;
- si lesdites données reçues sont incompatibles, rejette la demande d'enregistrement.

9. Procédé suivant l'une des revendications 7 à 8, dans lequel le message court est crypté par le terminal (30) de déclaration avant d'être émis.

10. Procédé suivant l'une des revendications 7 à 9, dans lequel des coordonnées géographiques établies par un récepteur de signaux de satellites de localisation couplé au terminal (30) de déclaration sont mémorisées par ledit terminal de déclaration, lors de la saisie du code d'identification (41) ou des données sur l'événement, et sont incorporées dans le message court envoyé au serveur.

11. Procédé suivant l'une des revendications 7 à 10, dans lequel l'événement est une naissance et dans lequel un prélèvement biologique d'un nouveau né auquel est fixé le support d'identification, tel qu'un bracelet d'identification (40), est déposé dans un évidement aménagé dans ledit support d'identification.

12. Procédé suivant l'une des revendications 7 à 11 dans lequel il est procédé à au moins une capture d'une ou plusieurs empreintes biométriques de l'individu auquel est attribué le support d'identification.

13. Procédé suivant l'une des revendications 7 à 12, dans lequel le terminal (30) de déclaration réalise une transmission du message court via un segment terminal (21) sans fil et, en cas d'échec de la transmission dudit message court, ledit terminal de déclaration émet à nouveau ledit message court lorsqu'il est détecté qu'une connexion au serveur est possible.

14. Procédé suivant l'une des revendications 7 à 13, dans lequel des supports d'identification nécessaires à la mise en œuvre dudit procédé sont, pour être déclarés authentiques après leur fabrication et préalablement à leur utilisation, identifiés dans un registre de supports d'identification authentiques dans lequel registre au moins un code d'identification (41) d'un support d'identification est apparié avec au moins une représentation numérique d'un élément authentifiant (42) incorporé audit support d'identification.

15. Procédé suivant l'une des revendications 8 ou 9 à 14, prise en combinaison avec la revendication 8, dans lequel les données reçues par le serveur (10) et copiées temporairement dans la base de données (11) d'état civil, associés à l'au moins un code d'identification, sont validées par un opérateur d'une station terminale (12) d'interrogation de ladite base de données sur présentation du support d'identification portant ledit au moins un code d'identification (41, 41a) si l'au moins un élément authentifiant porté par ledit support d'identification présente les attributs de structure des éléments authentifiant et si ledit élément authentifiant est conforme à l'au moins une représentation numérique dudit élément authentifiant appariée dans la base de données (11) audit au moins un code d'identification

16. Procédé suivant l'une des revendications 7 à 15, dans lequel les données reçues par le serveur (10) lors d'une déclaration sont relatives à un événement parmi : une naissance ; un mariage ou assimilé ; un divorce ou assimilé ; un changement de statut administratif; un décès.

17. Procédé suivant l'une des revendications 7 à 16 dans lequel, lorsque l'événement est relatif à un groupe de personnes, les données relatives à l'événement, enregistrées dans la base de données (11) des données d'état civil associées à un code d'identification, sont en outre associées, au moins temporairement en attente d'une étape de validation, à ou aux codes d'identification attribués à chacune des personnes dudit groupe connues dans ladite base de données.

## Patentansprüche

1. System (100) zur Anmeldung von Ereignissen, die sich auf Personen oder Personengruppen beziehen, zur Eintragung in ein digitalisiertes Personenstandsregister von Daten, die sich auf die Ereignisse und auf die Personen oder Personengruppen beziehen, und zur Ausfertigung von zertifizierten offiziellen Dokumenten (50), wobei das System einen Server (10) aufweist, wobei der Server eine Datenbank (11) der Personenstandsdaten der Personen einer Bevölkerung aufweist, die von dem Server verwaltet wird, aufweisend mindestens eine Endstelle (12) zur Abfrage der Datenbank (11) und zum Druck von offiziellen Dokumenten auf der Basis der in der Datenbank gespeicherten Daten, **dadurch gekennzeichnet, dass** es mindestens ein Anmeldeendgerät (30) und mindestens einen Identifikationsträger je in das Personenstandsregister eingetragenes oder einzutragendes Ereignis aufweist, und wobei in dem System:
a) jeder Identifikationsträger im Stadium seiner Herstellung aufweist:
- mindestens einen einzigartigen Identifikationscode (41, 41a, 41b);
- mindestens ein einzigartiges und nicht reproduzierbares authentifizierendes Element (42), ausgewählt aus den Markern, die eine einzigartige und nicht reproduzierbare dreidimensionale beobachtbare Struktur aufweisen, wobei die beobachtbare Struktur visuell und/oder elektronisch kontrollierbar ist, und wobei die Bildung der beobachtbaren Struktur aus einem chaotischen und zufallsbedingten Prozess resultiert, der im Material beobachtbare Heterogenitäten erzeugt, beispielsweise Marker, die in eine Polymermatrix eingeschlossene Blasen aufweisen, beispielsweise in eine Struktur eines Papiers eingeflochtene farbige Fasern;
- wobei der mindestens eine Identifikationscode und mindestens eine digitale Darstellung des mindestens einen authentifizierenden Elements vor Zuweisung zu einem Ereignis in einem Register (11a) authentischer Identifizierungsträger gepairt werden und die digitale Darstellung des authentifizierenden Elements ein Bild in digitaler Form des authentifizierenden Elements ist, wie es von einem Beobachter gesehen werden kann;
b) das mindestens eine Anmeldeendgerät (30) ausgelegt ist, um die Anmeldung von Ereignissen entfernt vom Registrierungsort durchzuführen und Erfassungsmittel des oder der Identifikationscodes aufweist, die auf dem Identifikationsträger vorhanden sind, Mittel zur Übertragung von Daten über ein Kommunikationsnetz (20) mit dem Server (10), und eine Softwareanwendung aufweist:
- zur Erfassung des mindestens einen Identifikationscodes (41, 41a, 41b);
- zur Eingabe der Daten, die sich auf ein Ereignis beziehen, das sich auf eine Person oder eine Personengruppe bezieht;
- zur Erstellung einer Kurzmitteilung, die den mindestens einen einzigartigen Identifikationscode aufweist und die eingegebenen Daten, die sich auf ein Ereignis beziehen, das sich auf eine Person oder eine Personengruppe bezieht, der der Identifikationsträger, der den mindestens einen einzigartigen Identifikationscode aufweist, zugewiesen wurde oder zuzuweisen ist;
- zur Übertragung der Kurzmitteilung an den Server (10) über das Kommunikationsnetz (20);
c) die mindestens eine Abfrageendstelle (12) eine Anwendung zur Herstellung von offiziellen Dokumenten (50) aufweist, in der mindestens eine visuelle Darstellung (42') des mindestens einen authentifizierenden Elements (42), dessen mindestens eine digitale Darstellung mit der mindestens einen einzigartigen Kennung im Register (11a) authentischer Identifikationsträger eines Identifikationsträgers, der einer Person oder einer Personengruppe zugewiesen ist, gepairt wird, in ein offizielles Dokument gedruckt wird, das sich auf den Personenstand der Person oder einer Person einer entsprechenden Personengruppe bezieht, und eine visuelle Erscheinung des authentifizierenden Elements (42) in allen ihren Details mit der gedruckten visuellen Darstellung (42') des authentifizierenden Elements übereinstimmt.

2. System nach Anspruch 1, wobei das mindestens eine Anmeldeendgerät (30) ein tragbares Endgerät ist, beispielsweise ein persönlicher Telefon-Rechner, das anhand eines drahtlosen Endgerätesegments (21) eines terrestrischen oder Satelliten-Funktelefonnetzes kommuniziert.

3. System nach einem der vorangehenden Ansprüche, wobei der mindestens eine Identifikationscode (41) des Identifikationsträgers durch eine auf dem Identifikationsträger lesbare alphanumerische Zeichenkette (41a) dargestellt und/oder in mindestens einer mit den optischen Lesemitteln (31) lesbaren grafischen Darstellung, beispielsweise in einem zweidimensionalen grafischen Code (QR-Code), enthalten ist und/oder in einem kontaktlos abfragbaren digitalen Speicher, der in den Identifikationsträger inkorporiert ist, enthalten ist.

4. System nach einem der vorangehenden Ansprüche, wobei der Identifikationsträger ein Identifikationsarmband (40) ist, das ein Verschlusssystem (43) aufweist, das nicht demontierbar ist, es sei denn, das Verschlusssystem wird zerstört.

5. System nach einem der vorangehenden Ansprüche, wobei der Identifikationsträger eine Ausnehmung aufweist, die mit einem flüssigkeitsabsorbierenden Material gefüllt ist und eine dichte Verschlussklappe der Ausnehmung aufweist.

6. System nach einem der vorangehenden Ansprüche, wobei das Anmeldeendgerät (30) eine Aufzeichnungsvorrichtung eines biometrischen Abdrucks aufweist.

7. Verfahren (100) zur Anmeldung von Ereignissen, die sich auf Personen oder Personengruppen beziehen, zur Eintragung in ein digitalisiertes Personenstandsregister von Daten, die sich auf die Ereignisse und auf die Personen oder Personengruppen beziehen, und zur Ausfertigung von zertifizierten offiziellen Dokumenten (50), wobei das Verfahren von einem System durchgeführt wird, das einen Server (10) aufweist, wobei der Server eine Datenbank (11) der Personenstandsdaten der Personen einer Bevölkerung aufweist, die von dem Server verwaltet wird, aufweisend mindestens eine Endstelle (12) zur Abfrage der Datenbank (11) und zum Druck von offiziellen Dokumenten auf der Basis der in der Datenbank gespeicherten Daten, mindestens ein Anmeldeendgerät (30) und mindestens einen Identifikationsträger je in das Personenstandsregister eingetragenes oder einzutragendes Ereignis, wobei das Verfahren die folgenden Schritte umfasst:
a) einen Schritt des Zuweisens eines Identifikationsträgers an die Person oder an mindestens einen Vertreter der Personengruppe bei einem Ereignis, das sich auf die Person oder die Personengruppe bezieht, wobei der Identifikationsträger im Stadium seiner Herstellung aufweist:
- mindestens einen einzigartigen Identifikationscode (41, 41a, 41b); und
- mindestens ein einzigartiges und nicht reproduzierbares authentifizierendes Element (42), ausgewählt aus den Markern, die eine einzigartige und nicht reproduzierbare dreidimensionale beobachtbare Struktur aufweisen, wobei die beobachtbare Struktur visuell und/oder elektronisch kontrollierbar ist, und wobei die Bildung der beobachtbaren Struktur aus einem chaotischen und zufallsbedingten Prozess resultiert, der im Material beobachtbare Heterogenitäten erzeugt, beispielsweise Marker, die in eine Polymermatrix eingeschlossene Blasen aufweisen, beispielsweise in eine Struktur eines Papiers eingeflochtene farbige Fasern;
- wobei der mindestens eine Identifikationscode und mindestens eine digitale Darstellung des mindestens einen authentifizierenden Elements vor Zuweisung zu einem Ereignis in einem Register (11a) authentischer Identifizierungsträger gepairt werden und die digitale Darstellung des authentifizierenden Elements ein Bild in digitaler Form des authentifizierenden Elements ist, wie es von einem Beobachter gesehen werden kann;
b) Anwenden des mindestens einen Anmeldeendgeräts (30), um die Anmeldung von Ereignissen entfernt vom Registrierungsort durchzuführen, und das Erfassungsmittel des oder der Identifikationscodes, die auf dem Identifikationsträger vorhanden sind, Mittel zur Übertragung von Daten über ein Kommunikationsnetz (20) mit dem Server (10), und eine Softwareanwendung aufweist, die eingerichtet ist, um die folgenden Schritte durchzuführen:
- Erfassen des mindestens einen Identifikationscodes (41, 41a, 41b);
- Eingabe der Daten, die sich auf ein Ereignis beziehen, das sich auf eine Person oder eine Personengruppe bezieht, und der persönlichen Daten, die sich auf die Person oder auf die Personen der Personengruppe beziehen;
- Erstellen einer Kurzmitteilung, die den mindestens einen einzigartigen Identifikationscode, die eingegebenen Daten, die sich auf das Ereignis beziehen, und die persönlichen Daten, die sich auf die Person oder auf die Personen der Personengruppe beziehen, aufweist;
- Übertragen der Kurzmitteilung an den Server (10) über das Kommunikationsnetz (20);
c) Drucken, in ein offizielles Dokument, das sich auf den Personenstand der Person oder einer Person der entsprechenden Personengruppe bezieht, durch eine Anwendung zur Herstellung von offiziellen Dokumenten (50) der mindestens einen Abfrageendstelle (12), neben den Daten des Personenstandsregisters, die bei dem entsprechenden Ereignis gemeldet werden, von mindestens einer visuellen Darstellung (42') des mindestens einen authentifizierenden Elements (42), von dem mindestens eine digitale Darstellung mit der mindestens einen einzigartigen Kennung im Register (11a) authentischer Identifikationsträger des Identifikationsträgers, der der Person oder dem Vertreter der Personengruppe zugewiesen ist, gepairt wird, und von dem eine visuelle Erscheinung in allen ihren Details mit der gedruckten visuellen Darstellung (42') des authentifizierenden Elements übereinstimmt.

8. Verfahren nach Anspruch 7, wobei der Server nach Erhalt der Kurzmitteilung aus der Kurzmitteilung die Daten und den mindestens einen Identifikationscode extrahiert, die Existenz und die Verfügbarkeit des empfangenen Identifikationscodes im Register (11a) der authentischen Identifizierungsträger überprüft und:
- wenn der Identifikationscode bekannt und verfügbar ist, der Server das Ereignis registriert und die empfangenen Daten in die Personenstandsdatenbank (11) kopiert, die dem mindestens einen Identifikationscode und der mindestens einen digitalen Darstellung des mindestens einen authentifizierenden Elements (42) zugewiesen sind, das mit dem mindestens einen Identifikationscode gepairt ist, dann den entsprechenden Identifikationscode als zugewiesen erklärt;
- wenn der Identifikationscode bekannt ist und zuvor als zugewiesen erklärt wurde, der Server überprüft, ob die empfangenen Daten mit dem oder den zuvor für den Identifikationscode registrierten Ereignissen kompatibel sind, dann:
- wenn die empfangenen Daten kompatibel sind, der Server temporär, in Erwartung eines Validierungsschritts, die empfangenen Daten in die Personenstandsdatenbank (11) kopiert, die dem mindestens einen Identifikationscode zugewiesen sind;
- wenn die empfangenen Daten inkompatibel sind, die Registrierungsanfrage abweist.

9. Verfahren nach einem der Ansprüche 7 bis 8, wobei die Kurzmitteilung vor dem Senden von dem Anmeldeendgerät (30) verschlüsselt wird.

10. Verfahren nach einem der Ansprüche 7 bis 9, wobei geografische Koordinaten, die von einem mit dem Anmeldeendgerät (30) gekoppelten Empfänger von Lokalisierungssatellitensignalen erstellt werden, von dem Anmeldeendgerät bei der Eingabe des Identifikationscodes (41) oder der Daten über das Ereignis gespeichert und in die an den Server geschickte Kurzmitteilung inkorporiert werden.

11. Verfahren nach einem der Ansprüche 7 bis 10, wobei das Ereignis eine Geburt ist und wobei eine biologische Probe eines Neugeborenen, an dem der Identifikationsträger wie ein Identifikationsarmband (40) befestigt ist, in einer Ausnehmung angeordnet wird, die in dem Identifikationsträger eingerichtet ist.

12. Verfahren nach einem der Ansprüche 7 bis 11, wobei mindestens eine Aufzeichnung von einer oder mehreren biometrischen Abdrücken der Person durchgeführt wird, der der Identifikationsträger zugewiesen ist.

13. Verfahren nach einem der Ansprüche 7 bis 12, wobei das Anmeldeendgerät (30) eine Übertragung der Kurzmitteilung über ein drahtloses Endgerätesegment (21) durchführt und bei einem Scheitern der Übertragung der Kurzmitteilung das Anmeldeendgerät die Kurzmitteilung erneut sendet, wenn festgestellt wird, dass eine Verbindung mit dem Server möglich ist.

14. Verfahren nach einem der Ansprüche 7 bis 13, wobei für die Durchführung des Verfahrens notwendigen Identifikationsträger nach ihrer Herstellung und vor ihrer Benutzung in einem Register authentischer Identifizierungsträger identifiziert werden, wobei in dem Register mindestens ein Identifikationscode (41) eines Identifikationsträgers mit mindestens einer digitalen Darstellung eines authentifizierenden Elements (42) gepairt wird, das in den Identifikationsträger inkorporiert ist.

15. Verfahren nach einem der Ansprüche 8 oder 9 bis 14, herangezogen in Kombination mit Anspruch 8, wobei die von dem Server (10) empfangenen und temporär in die Personenstandsdatenbank (11) kopierten Daten, die dem mindestens einen Identifikationscode zugewiesen sind, von einem Bediener einer Abfrageendstelle (12) der Datenbank bei Vorlage des Identifikationsträgers validiert werden, der den mindestens einen Identifikationscode (41, 41a) trägt, wenn das mindestens eine authentifizierende Element, das von dem Identifikationsträger getragen wird, die Strukturattribute der authentifizierenden Elemente trägt und wenn das authentifizierendes Element mit der mindestens einen digitalen Darstellung des authentifizierenden Elements übereinstimmt, die in der Datenbank (11) mit dem mindestens einen Identifikationscode gepairt ist.

16. Verfahren nach einem der Ansprüche 7 bis 15, wobei sich die vom Server (10) bei der Anmeldung empfangenen Daten auf ein Ereignis aus: einer Geburt; einer Eheschließung oder ähnliches; einer Scheidung oder ähnliches; einer Änderung des administrativen Status; einem Todesfall beziehen.

17. Verfahren nach einem der Ansprüche 7 bis 16, wobei, wenn sich das Ereignis auf eine Personengruppe bezieht, die Daten, die sich auf das Ereignis beziehen, die in der Datenbank (11) der Personenstandsdaten, die einem Identifikationscode zugewiesen sind, eingetragen sind, ferner mindestens temporär in Erwartung eines Validierungsschritts einem oder Identifikationscodes zugeordnet werden, die jeder der Personen der Gruppe zugewiesen sind, die in der Datenbank bekannt sind.

## Claims

1. System (100) for declaring events relative to individuals or to groups of individuals, recording in a computerised civil register data relative to said events and to said individuals or groups of individuals, and producing certified official documents (50), said system including a server (10), said server including a database (11) of the vital-record data of the individuals of a population managed by said server, including at least one terminal station (12) for querying the database (11) and printing official documents on the basis of the data memorised in said database, **characterised in that** it includes at least one declaration terminal (30) and at least one identification medium per event recorded or to be recorded in said civil register, and in which system:
- a) each identification medium includes during the stage of its manufacturing:
-- at least one unique identification code (41, 41a, 41b);
-- at least one unique and non-reproducible authenticating element (42) chosen from markers including a unique and non-reproducible three-dimensional observable structure, wherein said observable structure can be verified visually and/or electronically, and the formation of said observable structure results from a chaotic and random process producing observable heterogeneities in the material, for example markers including bubbles imprisoned in a polymer matric, for example coloured fibres interlaced into the structure of a paper;
-- said at least one identification code and at least one digital representation of the at least one authenticating element being, before being assigned to an event, paired in a register (11a) of authentic identification media, and said digital representation of the authenticating element is an image in digital form of the authenticating element as it can be seen by an observer;
- b) the at least one declaration terminal (30) is configured to carry out the declaration of events remotely from the recording location and includes means for acquiring the identification code(s) present on the identification medium, means for transmitting data via a network (20) for communication with the server (10), and includes a software application:
-- for acquiring the at least one identification code (41, 41a, 41b);
-- for entering data relative to an event relative to an individual or a group of individuals;
-- for creating a short message including the at least one unique identification code and the entered data relative to an event relative to an individual or a group of individuals to which the identification medium, including the at least one unique identification code, was or is to be assigned;
-- for transmitting said short message to the server (10) via the communication network (20);
- c) the at least one querying terminal station (12) includes an application for creating official documents (50) in which at least one visual representation (42') of the at least one authenticating element (42), at least one digital representation of which is paired with the at least one unique identifier in the register (11a) of authentic identification media, of an identification medium assigned to an individual or a group of individuals, is printed in an official document relative to the vital records of the individual or of an individual of the group of individuals considered, and a visual aspect of the authenticating element (42) conforms in all its details to the printed visual representation (42') of the authenticating element.

2. System according to claim 1, wherein the at least one declaration terminal (30) is a portable terminal, for example a personal smartphone, which communicates via a wireless terminal segment (21) of a terrestrial or satellite radiotelephony network.

3. System according to one of the previous claims, wherein the at least one identification code (41) of the identification medium is represented by a string of alphanumeric characters (41a) readable on the identification medium, and/or is contained in at least one graphic representation readable with the optical reading means (31), for example in a two-dimensional graphic code (QR-Code), and/or is contained in a digital memory that can be queried without contact incorporated into said identification medium.

4. System according to one of the previous claims, wherein the identification medium is an identification bracelet (40) that includes a closing system (43) that cannot be disassembled or can only by deteriorating said closing system.

5. System according to one of the previous claims, wherein the identification medium includes a recess filled with a liquid-absorbing material and includes a sealed closing shutter of said recess.

6. System according to one of the previous claims, wherein the declaration terminal (30) includes a device for capturing a biometric print.

7. Method (100) for declaring events relative to individuals or to groups of individuals, recording in a computerised civil register data relative to said events and to said individuals or groups of individuals, and producing certified official documents (50), said method being implemented by a system including a server (10), said server including a database (11) of the vital-record data of the individuals of a population managed by said server, including at least one terminal station (12) for querying the database (11) and printing official documents on the basis of the data memorised in said database, at least one declaration terminal (30) and at least one identification medium per event recorded or to be recorded in said civil register, said method comprising the following steps:
- a) a step of assigning an identification medium to said individual or to at least one representative of said group of individuals during an event relative to said individual or to said group of individuals, said identification medium including during the stage of its manufacturing:
-- at least one unique identification code (41, 41a, 41b); and
-- at least one unique and non-reproducible authenticating element (42) chosen from markers including a unique and non-reproducible three-dimensional observable structure, wherein said observable structure can be verified visually and/or electronically, and the formation of said observable structure results from a chaotic and random process producing observable heterogeneities in the material, for example markers including bubbles imprisoned in a polymer matric, for example coloured fibres interlaced into the structure of a paper;
-- said at least one identification code and at least one digital representation of the at least one authenticating element being, before being assigned to an event, paired in a register (11a) of authentic identification media, and said digital representation of the authenticating element is an image in digital form of the authenticating element as it can be seen by an observer;
- b) implementing the at least one declaration terminal (30) to carry out the declaration of events remotely with respect to the recording location, and including means for acquiring the identification code(s) present on the identification medium, means for transmitting data via a network (20) for communication with the server (10), and a software application arranged to carry out the following steps:
-- acquiring the at least one identification code (41, 41a, 41b);
-- entering the data relative to an event relative to an individual or a group of individuals, and the personal data relative to said individual or to the individuals of said group of individuals;
-- creating a short message including the at least one unique identification code, said entered data relative to the event and said personal data relative to said individual or to the individuals of said group of individuals;
-- transmitting said short message to the server (10) via the communication network (20);
- c) printing, in an official document relative to the vital records of the individual or of an individual of the group of individuals considered, by an application for creating official documents (50) of the at least one querying terminal station (12), in addition to the data from the civil register declared during the event considered, at least one visual representation (42') of the at least one authenticating element (42), at least one digital representation of which is paired with the at least one unique identifier in the register (11a) of authentic identification media, of said identification medium assigned to said individual or to said representative of said group of individuals, and a visual aspect of which conforms in all its details to the printed visual representation (42') of the authenticating element.

8. Method according to claim 7, wherein the server, after having received the short message, extracts from said short message said data and said at least one identification code, verifies the existence and the availability of the identification code received in the register (11a) of the authentic identification media and:
- if the identification code is known and available, the server creates a recording of the event and copies the data received into the vital-record database (11), associated with the at least one identification code and with the at least one digital representation of the at least one authenticating element (42) paired with said at least one identification code, then declares said corresponding identification code assigned;
- if the identification code is known and has been previously assigned, the server verifies whether the data received is compatible with the event(s) previously recorded for said identification code then:
-- if said received data is compatible, the server temporarily copies, while waiting for a validation step, the received data into the vital-record database (11), associated with the at least one identification code;
-- if said received data is incompatible, rejects the recording request.

9. Method according to one of claims 7 to 8, wherein the short message is encrypted by the declaration terminal (30) before being emitted.

10. Method according to one of claims 7 to 9, wherein the geographic coordinates established by a receiver of signals from location satellites coupled with the declaration terminal (30) are memorised by said declaration terminal, during the entering of the identification code (41) or of the data on the event, and are incorporated into the short message sent to the server.

11. Method according to one of claims 7 to 10, wherein the event is a birth and wherein a biological sample of a newborn to whom the identification medium, such as an identification bracelet (40), is attached is deposited in a recess arranged in said identification medium.

12. Method according to one of claims 7 to 11, wherein at least one capture of one or more biometric prints of the individual to whom the identification medium is assigned is carried out.

13. Method according to one of claims 7 to 12, wherein the declaration terminal (30) carries out a transmission of the short message via a wireless terminal segment (21) and, in the case of failure of the transmission of said short message, said declaration terminal emits said short message again when it is detected that a connection to the server is possible.

14. Method according to one of claims 7 to 13, wherein identification media necessary for the implementation of said method are, to be declared authentic after their manufacturing and before their use, identified in a register of authentic identification media in which register at least one identification code (41) of an identification medium is paired with at least one digital representation of an authenticating element (42) incorporated into said identification medium.

15. Method according to one of claims 8 or 9 to 14, taken in combination with claim 8, wherein the data received by the server (10) and temporarily copied into the vital-record database (11), associated with the at least one identification code, is validated by an operator of a terminal station (12) for querying said database upon presentation of the identification medium carrying said at least one identification code (41, 41a) if the at least one authenticating element carried by said identification medium presents the structural attributes of the authenticating elements and if said authenticating element conforms to the at least one digital representation of said authenticating element paired in the database (11) with said at least one identification code.

16. Method according to one of claims 7 to 15, wherein the data received by the server (10) during a declaration is relative to an event out of: a birth; a marriage or equivalent; a divorce or equivalent; a change in administrative status; a death.

17. Method according to one of claims 7 to 16, wherein, when the event is relative to a group of people, the data relative to the event, recorded in the database (11) of the vital-record data associated with an identification code, is further associated, at least temporarily while waiting for a validation step, with a or the identification codes assigned to each of the persons of said group known in said database.
